# EUROPEAN PATENT SPECIFICATION

(11) **EP 0 617 033 B1**
(45) Date of publication and mention of the grant of the patent: **12.08.1998**
(21) Application number: 94104098.2
(22) Date of filing: 16.03.1994
(51) Int. Cl.: C07D 405/04, C07D 239/54, C07D 405/10, A01N 43/54

(54) **Dihydrobenzofuran derivatives, their production and use**
Dihydrobenzofuranderivate, ihre Herstellung und Verwendung
Dérivés de dihydrobenzofuranne, leur préparation et leur utilisation

(30) Priority: 17.03.1993 JP 57216/93
(43) Date of publication of application: 28.09.1994
(73) Proprietor: Sumitomo Chemical Company, Limited, Chuo-ku Osaka 541-8550 (JP)
(72) Inventor: Takemura, Susumu, Takarazuka-shi, Hyogo-ken (JP); Takano, Minoru, Kameoka-shi, Kyoto-fu (JP); Kizawa, Satoru, Takarazuka-shi, Hyogo-ken (JP); Saito, Kazuo, Toyonaka-shi, Osaka-fu (JP)
(74) Representative: VOSSIUS & PARTNER

(56) References cited:
- EP-A- 0 255 047
- EP-A- 0 271 170
- EP-A- 0 476 697
- EP-A- 0 517 181
- WO-A-93/14073

## Description

The present invention relates to novel dihydrobenzofuran derivatives and herbicidal compositions containing them as active ingredients.

EP-A-0 476 697 discloses uracil derivatives, their production and use as herbicides.

WO 93/14073 which is prior art under Art 54(3) EPC for the contracting states DE, ES, FR, GB and IT describes pyrimidin compounds useful as herbicides.

It is well known that certain kinds of substituted dihydrobenzofuran derivatives can be used as active ingredients of herbicides (see, e.g., USP 4,881,967).

These compounds, however, have insufficient herbicidal activity and poor selectivity between crop plants and weeds, and it cannot always be said that they are satisfactory for active ingredients of herbicides.

Under these circumstances, the present inventors have intensively studied various compounds. As the result, they have found that particular kinds of dihydrobenzofuran derivatives have excellent herbicidal activity and exhibit excellent selectivity between crop plants and weeds, thereby completing the present invention.

Thus, the present invention provides novel dihydrobenzofuran derivatives of the formula: 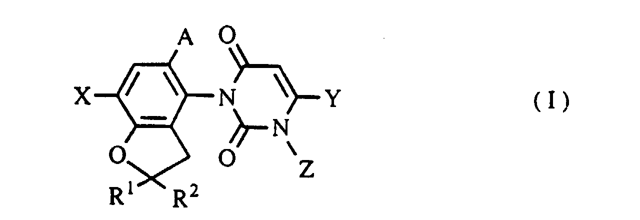 wherein A is hydrogen, fluorine or chlorine; X is hydrogen, fluorine, chlorine or bromine; Y is methyl optionally substituted with one or more halogen atoms; Z is methyl or amino; R¹ is hydrogen or C₁-C₆ alkyl; and R² is C₁-C₆ alkyl, C₁-C₆ haloalkyl, C₁-C₆ hydroxyalkyl, C₁-C₆ alkoxy(C₁-C₆)alkyl, C₁-C₆ alkoxy(C₁-C₆)alkoxy(C₁-C₆)-alkyl, C₁-C₇ acyloxy(C₁-C₆)alkyl, carboxyl, C₁-C₆ alkoxycarbonyl, C₁-C₆ haloalkoxycarbonyl, C₃-C₆ cycloalkoxycarbonyl, C₂-C₆ alkynyloxycarbonyl, aminocarbonyl, C₁-C₆ alkylaminocarbonyl or phenylaminocarbonyl having a phenyl group optionally substituted with C₁-C₆ alkyl, C₁-C₆ alkoxy, C₁-C₆ haloalkyl, C₁-C₆ haloalkoxy, halogen, nitro, cyano or C₁-C₆ alkylthio.

For the contracting states DE, ES, FR, GB and IT
X must be different from chlorine and bromine if R¹ is hydrogen and A is fluorine or chlorine.

On this basis the following description relates to all designated states.

In the formula (I) as depicted above, examples of the acyl group are C₁-C₆ alkylcarbonyl, C₁-C₆ haloalkylcarbonyl, C₃-C₆ cycloalkylcarbonyl or benzoyl, and examples of the halogen atom are fluorine, chlorine and bromine.

The present invention also provides a herbicidal composition comprising compound (I) as an active ingredient and a method of exterminating unfavorable weeds by applying compound (I) to an area where the unfavorable weeds grow or will grow.

The following will describe some production processes for the compound (I).

### <Production Process (a)>

The compound (I) wherein R² is C₁-C₆ alkyl can be produced by subjecting, to intramolecular cyclization, a compound of the formula: 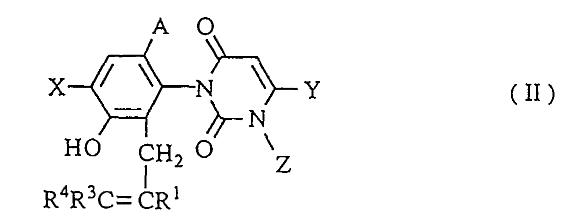 wherein R³ and R⁴ are the same or different and are hydrogen or C₁-C₆ alkyl with the proviso that the total number of carbon atoms in the R³ and R⁴ is not greater than 6, and A, X, Y, Z and R¹ are each as defined above.

The reaction is usually carried out without any solvent or in a solvent in the presence or absence of a catalyst at a temperature of 0° to 250°C, preferably 20° to 200°C, for a period of 0.5 to 24 hours. The catalyst is used in an amount of 0.01 to 0.5 moles to one mole of the compound (II).

Examples of the solvent are aliphatic hydrocarbons such as petroleum ether and hexane; aromatic hydrocarbons such as toluene and xylene; halogenated hydrocarbons such as chloroform, 1,2-dichloroethane, chlorobenzene and o-dichlorobenzene; ethers such as diethyl ether, diisopropyl ether, 1,4-dioxane, tetrahydrofuran and ethylene glycol dimethyl ether; ketones such as acetone, methyl isobutyl ketone and cyclohexanone; fatty acids such as formic acid and acetic acid; alcohols such as methanol, ethanol and ethylene glycol; nitro compounds such as nitroethane and nitrobenzene; nitriles such as acetonitrile and isobutyronitrile; acid amides such as formamide, N,N-dimethylformamide and acetamide; sulfur compounds such as dimethyl sulfoxide and sulforane; and water. These solvents may be used alone or in combination.

Examples of the catalyst are sulfonic acids such as p-toluenesulfonic acid and methanesulfonic acid; sulfonates such as pyridinium-p-toluenesulfonate; and mineral acids such as sulfonic acid and hydrochloric acid.

After completion of the reaction, the reaction mixture is subjected to ordinary post-treatment. For example, the reaction mixture is poured into water and the precipitated crystals are filtered by filtration, or alternatively the reaction mixture is extracted with an organic solvent and concentrated. If necessary , any purification such as chromatography, distillation or recrystallization, thus obtaining the desired compound.

The compound (II) can be produced by reacting a compound of the formula: 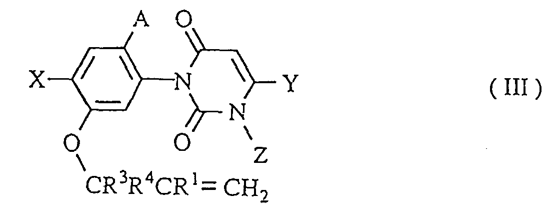 wherein A, X, Y, Z, R¹, R³ and R⁴ are each as defined above, without any solvent or in an solvent at a temperature of 20° to 300°C, preferably 100° to 250°C, for a period of 0.5 to 48 hours.

Examples of the solvent are aliphatic hydrocarbons such as petroleum ether and hexane; aromatic hydrocarbons such as toluene, xylene and m-isobutylbenzene; halogenated hydrocarbons such as chloroform, 1,2-dichloroethane, chlorobenzene and o-dichlorobenzene; ethers such as diethyl ether, diisopropyl ether, 1,4-dioxane, tetrahydrofuran and ethylene glycol dimethyl ether; ketones such as acetone, methyl isobutyl ketone and cyclohexanone; fatty acids such as formic acid and acetic acid; alcohols such as methanol, ethanol and ethylene glycol; esters such as ethyl acetate and diethyl carbonate; nitro compounds such as nitroethane and nitrobenzene; nitriles such as acetonitrile and isobutyronitrile; tertiary amines such as pyridine, triethylamine, N,N-dimethylaniline, N,N-diethylaniline and N-methylmorpholine; acid amides such as formamide, N,N-di-methylformamide and acetamide; sulfur compounds such as dimethyl sulfoxide and sulforane; and water. These solvents may be used alone or in combination.

After completion of the reaction, the reaction mixture is subjected to ordinary post-treatment. For example, the reaction mixture is poured into water and the precipitated crystals are filtered by filtration, or alternatively the reaction mixture is extracted with an organic solvent and concentrated. If necessary , any purification such as chromatography, distillation or recrystallization, thus obtaining the desired compound.

The compound (III) can be produced according to the method as described in JP-A-63-41466 and EP-A-517181.

### <Production Process (b)>

The compound (I) wherein R² is C₁-C₆ hydroxyalkyl can be produced by reacting the compound (II), which is the starting material of the production process (a), with an epoxidizing agent (the first step) and then subjecting the product to cyclization in the presence of a catalyst (the second step), if necessary, as depicted in the following scheme: 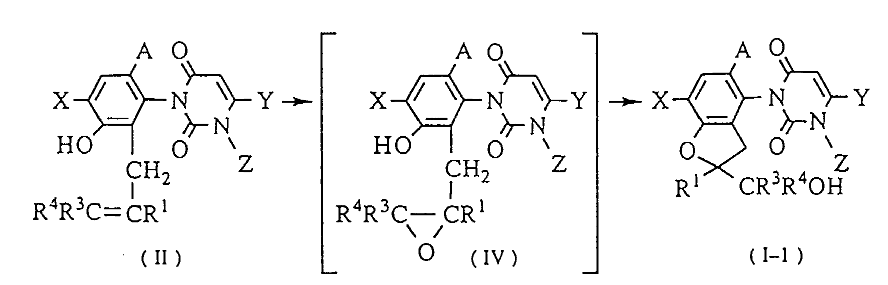 wherein A, X, Y, Z, R¹, R³ and R⁴ are each as defined above.

The first step is usually carried out in a solvent at a temperature of -20° to 150°C, preferably 0° to 80°C, for a period of 0.5 to 24 hours. The epoxidizing agent is used in an amount of 1 to 5 moles to one mole of the compound (II).

Examples of the solvent which can be used in the first step are aliphatic hydrocarbons such as petroleum ether and hexane; fatty acids such as formic acid and acetic acid; and halogenated hydrocarbons such as methylene chloride, chloroform and 1,2-dichloroethane. Examples of the epoxidizing agent are peracids such as perbenzoic acid, m-chloroperbenzoic acid, peracetic acid and trifluoroperacetic acid.

After completion of the reaction, the reaction mixture is treated with a reducing agent such as aqueous sodium thiosulfate or aqueous sodium hydrogensulfite, and then subjected to ordinary post-treatments such as extraction with an organic solvent and concentration, after which any purification is employed, if necessary, such as chromatography or recrystallization, thus obtaining the compound (IV) or the desired compound (I-1) which is formed from the compound (IV) by direct cyclization.

The second step is usually carried out in a solvent in the presence or absence of a catalyst at a temperature of -20° to 250°C, preferably 0° to 200°C, for a period of a moment to 24 hours. The catalyst is used in an amount of a catalytic amount to five moles to one mole of the compound (IV).

Examples of the solvent which can be used in the second step are aliphatic hydrocarbons such as petroleum ether and hexane; aromatic hydrocarbons such as toluene and xylene; halogenated hydrocarbons such as chloroform, 1,2-dichloroethane, chlorobenzene and o-dichlorobenzene; ethers such as diethyl ether, diisopropyl ether, 1,4-dioxane, tetrahydrofuran and ethylene glycol dimethyl ether; ketones such as acetone, methyl isobutyl ketone and cyclohexanone; fatty acids such as formic acid and acetic acid; alcohols such as methanol, ethanol and ethylene glycol; esters such as ethyl acetate and diethyl carbonate; nitro compounds such as nitroethane and nitrobenzene; nitriles such as acetonitrile and isobutyronitrile; tertiary amines such as pyridine, triethylamine, N,N-dimethylaniline, N,N-diethylaniline and N-methylmorpholine; acid amides such as formamide, N,N-dimethylformamide and acetamide; sulfur compounds such as dimethyl sulfoxide and sulforane; and water. These solvents may be used alone or in combination.

Depending upon the kind of solvent used, various catalysts may be used, examples of which are sulfonic acids such as p-toluenesulfonic acid and methanesulfonic acid; sulfonates such as pyridinium-p-toluenesulfonate; mineral acids such as sulfuric acid, hydrochloric acid and hydroperchloric acid; Lewis acids such as boron trifluoride diethyl etherate and zinc chloride; inorganic bases such as potassium carbonate, sodium hydroxide and potassium hydroxide; and metal hydrides such as sodium hydride and potassium hydride.

After completion of the reaction, the reaction mixture is subjected to ordinary post-treatment. For example, the reaction mixture is poured into water and the precipitated crystals are filtered by filtration, or alternatively the reaction mixture is extracted with an organic solvent and concentrated. If necessary, any purification such as chromatography, distillation or recrystallization, thus obtaining the desired compound.

### <Production Process (c)>

The compound (I) wherein R² is C₁-C₆ alkoxy(C₁-C₆)alkyl or C₁-C₆ alkoxy (C₁-C₆)alkoxy(C₁-C₆)alkyl can be produced by reacting the compound (I-1), which is produced by the production process (b) as described above, with a compound of the formula:

R⁵J (V)

wherein R⁵ is C₁-C₆ alkyl or C₁-C₆ alkoxy(C₁-C₆)alkyl, and J is halogen, methanesulfonyloxy or p-toluenesulfonyloxy.

The reaction is usually carried out in a solvent in the presence of a base at a temperature of -20°C to the reflux temperature of a solvent when used, for a period of a moment to 24 hours. The compound (V) and the base are used in the respective amounts of 1 to 10 moles to one mole of the compound (I-1).

Examples of the solvent are aliphatic hydrocarbons such as petroleum ether and hexane; aromatic hydrocarbons such as toluene and xylene; halogenated hydrocarbons such as chloroform, 1,2-dichloroethane, chlorobenzene and o-dichlorobenzene; ethers such as diethyl ether, diisopropyl ether, 1,4-dioxane, tetrahydrofuran and ethylene glycol dimethyl ether; ketones such as acetone, methyl isobutyl ketone and cyclohexanone; esters such as ethyl acetate and diethyl carbonate; nitro compounds such as nitroethane and nitrobenzene; nitriles such as acetonitrile and isobutyronitrile; tertiary amines such as pyridine, triethylamine, N,N-dimethylaniline, N,N-diethylaniline and N-methylmorpholine; acid amides such as formamide, N,N-dimethylformamide and acetamide; and sulfur compounds such as dimethyl sulfoxide and sulforane. These solvents may be used alone or in combination.

Examples of the base are inorganic bases such as potassium carbonate, sodium hydroxide and potassium hydroxide; metal hydrides such as sodium hydride and potassium hydride; and organic bases such as triethylamine, diisopropylethylamine, pyridine and 4-dimethylaminopyridine.

After completion of the reaction, the reaction mixture is subjected to ordinary post-treatment. For example, the reaction mixture is poured into water and the precipitated crystals are filtered by filtration, or alternatively the reaction mixture is extracted with an organic solvent and concentrated. If necessary, any purification such as chromatography, distillation or recrystallization, thus obtaining the desired compound.

### <Production Process (d)>

The compound (I) wherein R² is C₁-C₇ acyloxyalkyl can be produced by reacting the compound (I-1), which is produced by the production process (b), with a compound of the formula:

R⁶G (VI)

wherein R⁶ is C₁-C₇ acyl and G is chlorine or bromine, or a compound of the formula:

(R⁶)₂O (VII)

wherein R⁶ is as defined above.

The reaction is usually carried out without any solvent or in a solvent in the presence of a base at a temperature of -20° to 200°C, preferably 0°C to the reflux temperature of a solvent when used, or preferably 0° to 100°C when no solvent is used, for a period of a moment to 24 hours. The compound (VI) or (VII) and the base are used in the respective amounts of one mole to in large excess to one mole of the compound (I-1).

Examples of the solvent are aliphatic hydrocarbons such as petroleum ether and hexane; aromatic hydrocarbons such as toluene and xylene; halogenated hydrocarbons such as chloroform, 1,2-dichloroethane, chlorobenzene and o-dichlorobenzene; ethers such as diethyl ether, diisopropyl ether, 1,4-dioxane, tetrahydrofuran and ethylene glycol dimethyl ether; ketones such as acetone, methyl isobutyl ketone and cyclohexanone; esters such as ethyl acetate and diethyl carbonate; nitro compounds such as nitroethane and nitrobenzene; nitriles such as acetonitrile and isobutyronitrile; tertiary amines such as pyridine, triethylamine, N,N-dimethylaniline, N,N-diethylaniline and N-methylmorpholine; acid amides such as formamide, N,N-dimethylformamide and acetamide; and sulfur compounds such as dimethyl sulfoxide and sulforane. These solvents may be used alone or in combination.

Examples of the base are inorganic bases such as potassium carbonate, sodium hydroxide and potassium hydroxide; metal hydrides such as sodium hydride and potassium hydride; and organic bases such as triethylamine, diisopropylethylamine, pyridine and 4-dimethylaminopyridine.

After completion of the reaction, the reaction mixture is subjected to ordinary post-treatment. For example, the reaction mixture is concentrated; the reaction mixture is poured into water and the precipitated crystals are filtered by filtration; or the reaction mixture is extracted with an organic solvent and concentrated. If necessary, any purification such as chromatography or recrystallization, thus obtaining the desired compound.

The compound (I) wherein R² is acyloxyalkyl can be produced by reacting the compound (I-1), which is produced by the production process (b), with a compound of the formula:

R⁶-OH (VII-2)

wherein R⁶ is as defined above.

The reaction is usually carried out without any solvent or in a solvent in the presence of an acid or a condensing agent at a temperature of 0° to 200°C, preferably 10°C to the reflux temperature of a solvent when used, or preferably 10° to 100°C when no solvent is used, for a period of a moment to 24 hours. The compound (VII-2) is used in an amount of one mole to in large excess to one mole of the compound (I-1). The acid is used in a catalytic amount or up to the amount of one mole to one mole of the compound (I-1). The condensing agent is used in an amount of 1 to 5 moles to one mole of the compound (I-1).

Examples of the solvent are aliphatic hydrocarbons such as petroleum ether and hexane; aromatic hydrocarbons such as toluene and xylene; halogenated hydrocarbons such as chloroform, 1,2-dichloroethane, chlorobenzene and o-dichlorobenzene; ethers such as diethyl ether, diisopropyl ether, 1,4-dioxane, tetrahydrofuran and ethylene glycol dimethyl ether; ketones such as acetone, methyl isobutyl ketone and cyclohexanone; esters such as ethyl acetate and diethyl carbonate; nitro compounds such as nitroethane and nitrobenzene; nitriles such as acetonitrile and isobutyronitrile; tertiary amines such as pyridine, triethylamine, N,N-dimethylaniline, N,N-diethylaniline and N-methylmorpholine; acid amides such as formamide, N,N-dimethylformamide and acetamide; and sulfur compounds such as dimethyl sulfoxide and sulforane. These solvents may be used alone or in combination.

Examples of the acid are sulfonic acids such as p-toluenesulfonic acid and methanesulfonic acid; sulfonates such as pyridinium-p-toluenesulfonate; and mineral acids such as sulfuric acid and hydrochloric acid.

Examples of the condensing agent are N,N'-disubstituted carbodiimide such as N,N'-dicyclohexylcarbodiimide; 2,4,6-trimethylbenzenesulfonyl chloride and N,N'-carbonyldiimidazole.

After completion of the reaction, the reaction mixture is subjected to ordinary post-treatment. For example, the reaction mixture is concentrated; the reaction mixture is poured into water and the precipitated crystals are filtered by filtration; or the reaction mixture is extracted with an organic solvent and concentrated. If necessary, any purification such as chromatography or recrystallization, thus obtaining the desired compound.

### <Production Process (e)>

The compound (I) wherein R² is carboxyl can be produced by oxidizing the compound (I-1) wherein R³ and R⁴ are both hydrogen with an oxidizing agent.

The reaction is usually carried out in a solvent at a temperature of -80° to 100°C, preferably 0° to 50°C, for a period of 0.5 to 12 hours. The oxidizing agent is used in an amount of one mole to in large excess to one mole of the compound (I-1).

Examples of the oxidizing agent are permanganates such as potassium permanganate; chromic acids such as chromium trioxide/sulfuric acid, potassium dichromate and pyridinium dichromate; and oxygen gas.

Depending upon the kind of oxidizing agent used, various solvents may be used, examples of which are aliphatic hydrocarbons such as petroleum ether and hexane; aromatic hydrocarbons such as toluene and xylene; halogenated hydrocarbons such as chloroform, 1,2-dichloroethane, chlorobenzene and o-dichlorobenzene; ketones such as acetone, methyl isobutyl ketone and cyclohexanone; fatty acids such as formic acid and acetic acid; esters such as ethyl acetate and diethyl carbonate; acid amides such as formamide, N,N-dimethylformamide and acetamide; sulfur compounds such as dimethyl sulfoxide and sulforane; and water. These solvents may be used alone or in combination.

After completion of the reaction, the reaction mixture is subjected to ordinary post-treatment. For example, the reaction mixture is poured into water and the precipitated crystals are filtered by filtration, or alternatively the reaction mixture is extracted with an organic solvent and concentrated. If necessary, any purification such as chromatography, distillation or recrystallization, thus obtaining the desired compound.

### <Production Process (f)>

The compound (I) wherein R² is C₁-C₆ alkoxycarbonyl, C₁-C₆ haloalkoxycarbonyl, C₃-C₆ cycloalkoxycarbonyl or C₂-C₆ alkynyloxycarbonyl can be produced by reacting the compound (I) wherein R² is carboxyl, which is produced by the production process (e), with a compound of the formula:

R⁷-OH (VIII)

wherein R⁷ is C₁-C₆ alkyl, C₁-C₆ haloalkyl, C₁-C₆ cycloalkyl or C₂-C₆ alkynyl.

The reaction is usually carried out without any solvent or in a solvent in the presence of an acid at a temperature of 20° to 200°C, preferably 60° to 120°C, for a period of 0.5 to 24 hours. The compound (VIII) and the acid are used in amounts of one mole to in large excess and a catalytic amount to 0.5 moles, respectively, to one mole of the compound (I) wherein R² is carboxyl.

Examples of the acid are mineral acids such as sulfuric acid and hydrochloric acid; and sulfonic acids such as p-toluenesulfonic acid.

Examples of the solvent are aliphatic hydrocarbons such as petroleum ether and hexane; aromatic hydrocarbons such as toluene and xylene; halogenated hydrocarbons such as chloroform, 1,2-dichloroethane, chlorobenzene and o-dichlorobenzene; ethers such as diethyl ether, diisopropyl ether, 1,4-dioxane, tetrahydrofuran and ethylene glycol dimethyl ether; acid amides such as formamide, N,N-dimethylformamide and acetamide; and sulfur compounds such as dimethyl sulfoxide and sulforane. These solvents may be used alone or in combination.

After completion of the reaction, the reaction mixture is subjected to ordinary post-treatment. For example, the reaction mixture is poured into water and the precipitated crystals are filtered by filtration, or alternatively the reaction mixture is extracted with an organic solvent and concentrated. If necessary, any purification such as chromatography, distillation or recrystallization, thus obtaining the desired compound.

The compound (I) wherein R² is alkoxycarbonyl, haloalkoxycarbonyl, cycloalkoxycarbonyl or alkynyloxycarbonyl can be produced by converting the compound (I) wherein R² is carboxyl, which is produced by the production process (e), into its activated reactive derivative according to a chemically acceptable method and then reacting this derivative with the compound (VIII).

For the activation, various methods can be used, such as those using phosgene, oxalyl chloride, thionyl chloride, phosphorous oxychloride, N,N'-carbonyldiimidazole, N,N'-dicyclohexylcarbodiimide or 2,4,6-trimethylbenzenesulfonyl chloride.

The reaction of the reactive derivative with the compound (VIII) is usually carried out in a solvent in the presence or absence of a base at a temperature of -20° to 100°C for a period of a moment to 24 hours. The compound (VIII) is used in an amount of one mole to in large excess to one mole of the compound (I) wherein R² is carboxyl. The base is used in an amount of 1 to 5 moles to one mole of the compound (I) wherein R² is carboxyl.

Examples of the solvent are aliphatic hydrocarbons such as petroleum ether and hexane; aromatic hydrocarbons such as toluene and xylene; halogenated hydrocarbons such as chloroform, 1,2-dichloroethane, chlorobenzene and o-dichlorobenzene; ethers such as diethyl ether, diisopropyl ether, 1,4-dioxane, tetrahydrofuran and ethylene glycol dimethyl ether; ketones such as acetone, methyl isobutyl ketone and cyclohexanone; nitro compounds such as nitroethane and nitrobenzene; nitriles such as acetonitrile and isobutyronitrile; tertiary amines such as pyridine, triethylamine, N,N-dimethylaniline, N,N-diethylaniline and N-methylmorpholine; acid amides such as formamide, N,N-dimethylformamide and acetamide; and sulfur compounds such as dimethyl sulfoxide and sulforane. These solvents may be used alone or in combination.

Examples of the base are inorganic bases such as potassium carbonate, sodium hydroxide and potassium hydroxide; and tertiary amines such as pyridine and triethylamine.

After completion of the reaction, the reaction mixture is subjected to ordinary post-treatment. For example, the reaction mixture is poured into water and the precipitated crystals are filtered by filtration, or alternatively the reaction mixture is extracted with an organic solvent and concentrated. If necessary, any purification such as chromatography, distillation or recrystallization, thus obtaining the desired compound.

### <Production Process (g)>

The compound (I) wherein R² is aminocarbonyl, C₁-C₆ alkylaminocarbonyl or phenylaminocarbonyl having a phenyl group optionally substituted with C₁-C₆ alkyl, C₁-C₆ alkoxy, C₁-C₆ haloalkyl, C₁-C₆ haloalkoxy, halogen, nitro, cyano or C₁-C₆ thioalkyl can be produced by converting the compound (I) wherein R² is carboxyl, which is produced by the production process (e), into its reactive derivative according to the production process (f) as described above and then reacting this derivative with a compound of the formula:

R⁸R⁹NH (IX)

wherein R⁸ and R⁹ are the same or different and are independently hydrogen, C₁-C₆ alkyl or phenyl optionally substituted with C₁-C₆ alkyl, C₁-C₆ alkoxy, C₁-C₆ haloalkyl, C₁-C₆ haloalkoxy, halogen, nitro, cyano or C₁-C₆ thioalkyl.

The reaction is usually carried out in a solvent at a temperature of 0° to 100°C, preferably 5° to 80°C, for a period of a moment to 24 hours. The compound (IX) is used in an amount of one mole to in large excess to one mole of the compound (I) wherein R² is carboxyl.

Examples of the solvent are aliphatic hydrocarbons such as petroleum ether and hexane; aromatic hydrocarbons such as toluene and xylene; halogenated hydrocarbons such as chloroform, 1,2-dichloroethane, chlorobenzene and o-dichlorobenzene; ethers such as diethyl ether, diisopropyl ether, 1,4-dioxane, tetrahydrofuran and ethylene glycol dimethyl ether; ketones such as acetone, methyl isobutyl ketone and cyclohexanone; esters such as ethyl acetate and diethyl carbonate; nitro compounds such as nitroethane and nitrobenzene; nitriles such as acetonitrile and isobutyronitrile; tertiary amines such as pyridine, triethylamine, N,N-dimethylaniline, N,N-diethylaniline and N-methylmorpholine; acid amides such as formamide, N,N-dimethylformamide and acetamide; and sulfur compounds such as dimethyl sulfoxide and sulforane. These solvents may be used alone or in combination.

After completion of the reaction, the reaction mixture is subjected to ordinary post-treatment. For example, the reaction mixture is poured into water and the precipitated crystals are filtered by filtration, or alternatively the reaction mixture is extracted with an organic solvent and concentrated. If necessary, any purification such as chromatography, distillation or recrystallization, thus obtaining the desired compound.

### <Production Process (h)>

The compound (I) wherein R² is C₁-C₆ haloalkyl can be produced by halogenating the compound (I-1), which is produced by the production process (b), with a halogenating agent.

The reaction is usually carried out without any solvent or in a solvent at a temperature of -20° to 200°C, preferably 0° to 150°C, for a period of 0.5 to 24 hours. The halogenating agent is used in an amount of 1 to 10 moles to one mole of the compound (I-1).

Examples of the solvent are halogenated hydrocarbons such as dichloromethane, chloroform and methylene chloride; aromatic hydrocarbons such as toluene and xylene; and ethers such as diethyl ether and tetrahydrofuran.

Examples of the halogenating agent are fluorinating agents such as diethylaminosulfur trifluoride; chlorinating agents such as carbon tetrachloride/triphenylphosphine; and brominating agents such as carbon tetrabromide/triphenylphosphine.

After completion of the reaction, the reaction mixture is subjected to ordinary post-treatment. For example, the reaction mixture is poured into water and the precipitated crystals are filtered by filtration, or alternatively the reaction mixture is extracted with an organic solvent and concentrated. If necessary , any purification such as chromatography, distillation or recrystallization, thus obtaining the desired compound.

According to any one of the above production processes (a) to (h), various compounds of the formula (I) are obtained as show in Table 1 where the symbols (n) and (c) denote normal chain alkyl and cycloalkyl groups, respectively.

Compounds (101) - (138), (182) and (184) - (189) of Table 1 below are only relevant for the contracting states AT, CH, LI and GR.

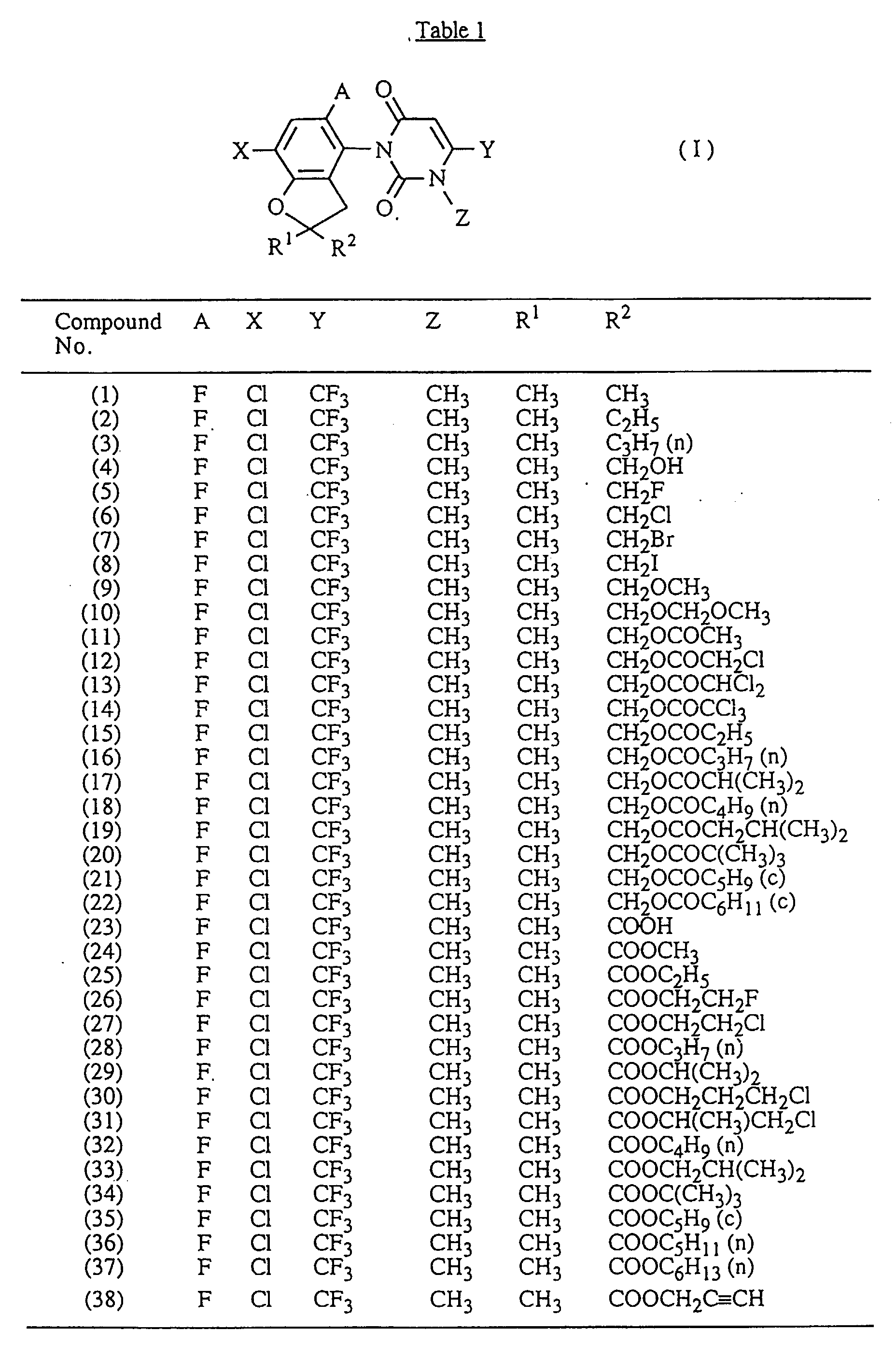

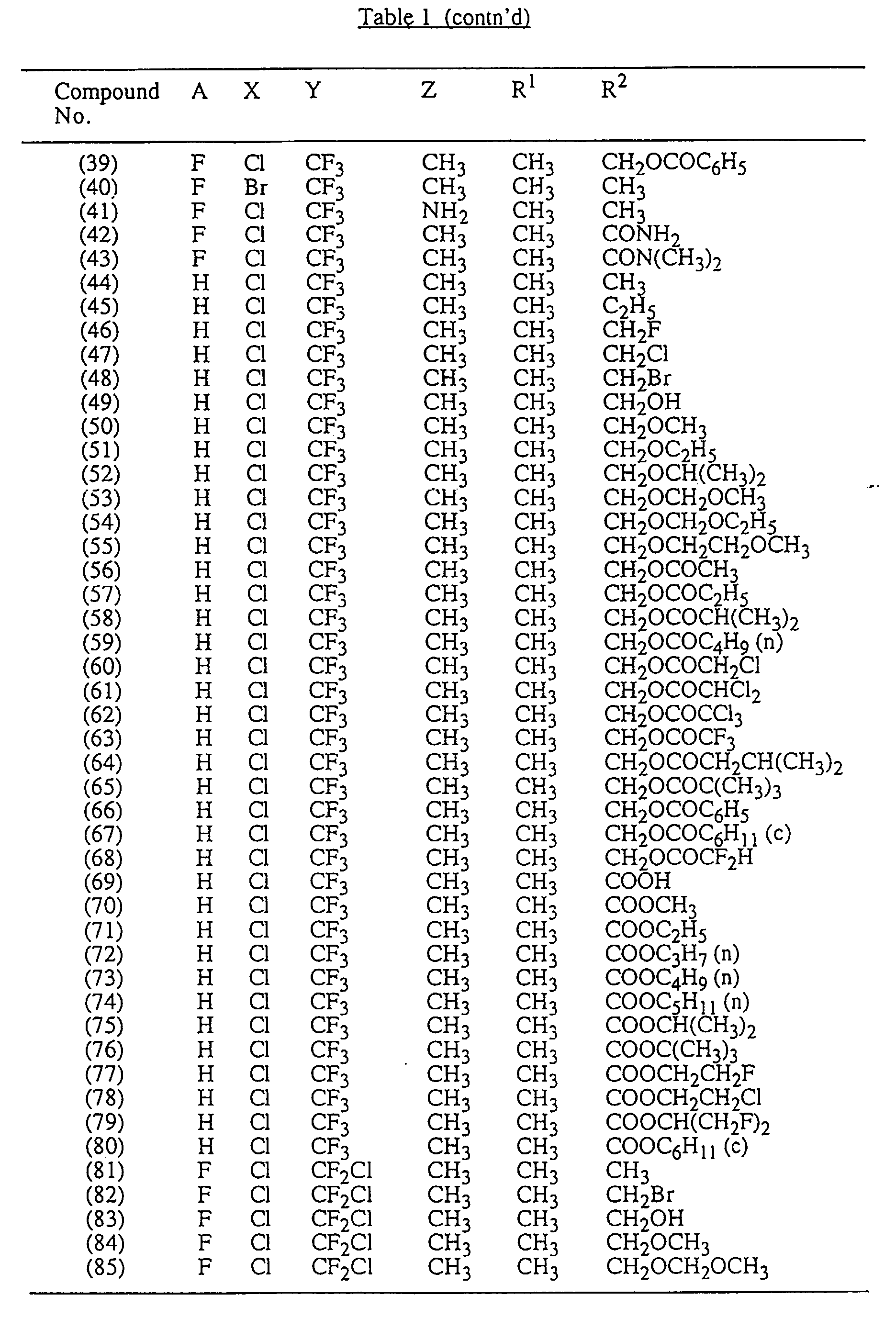

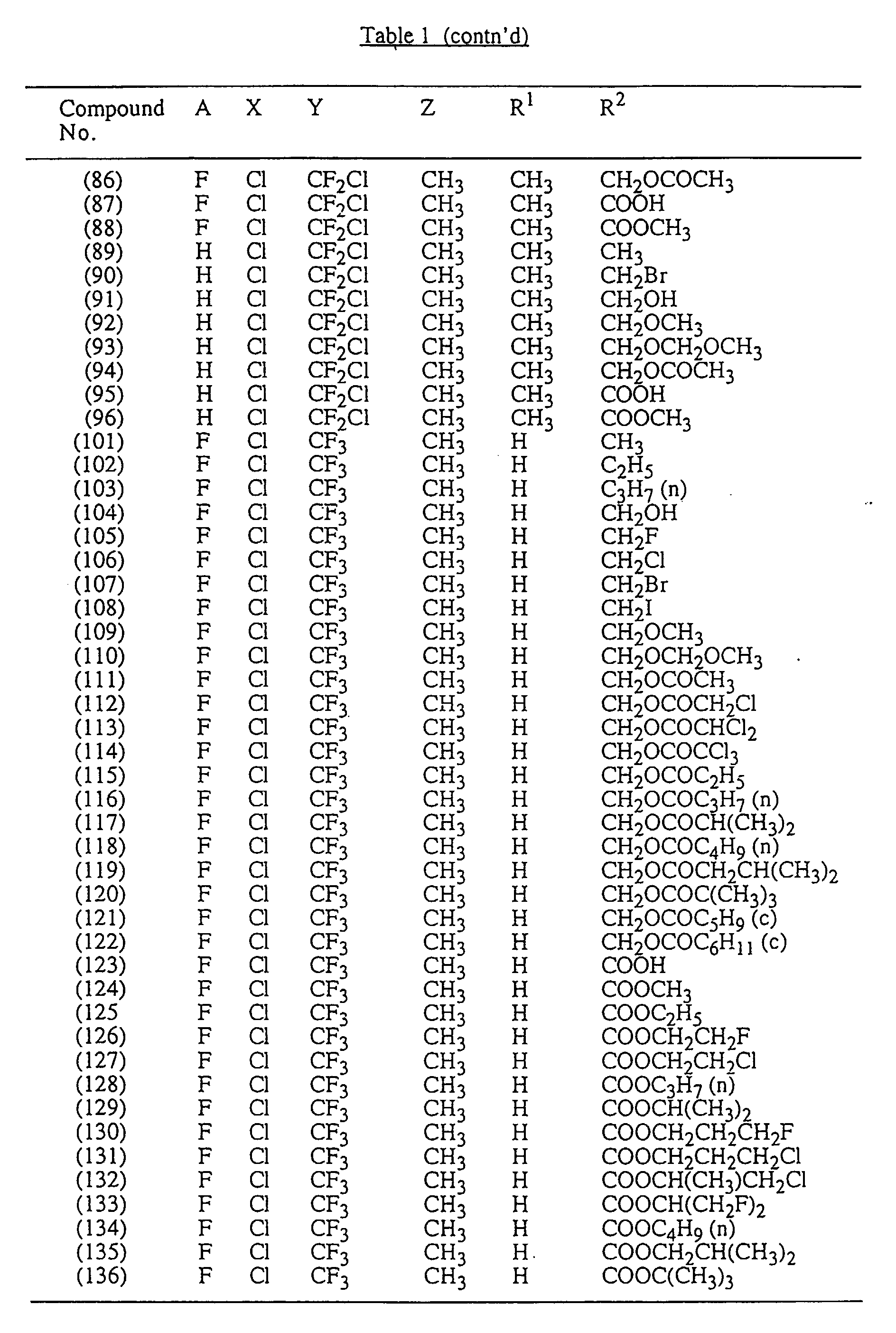

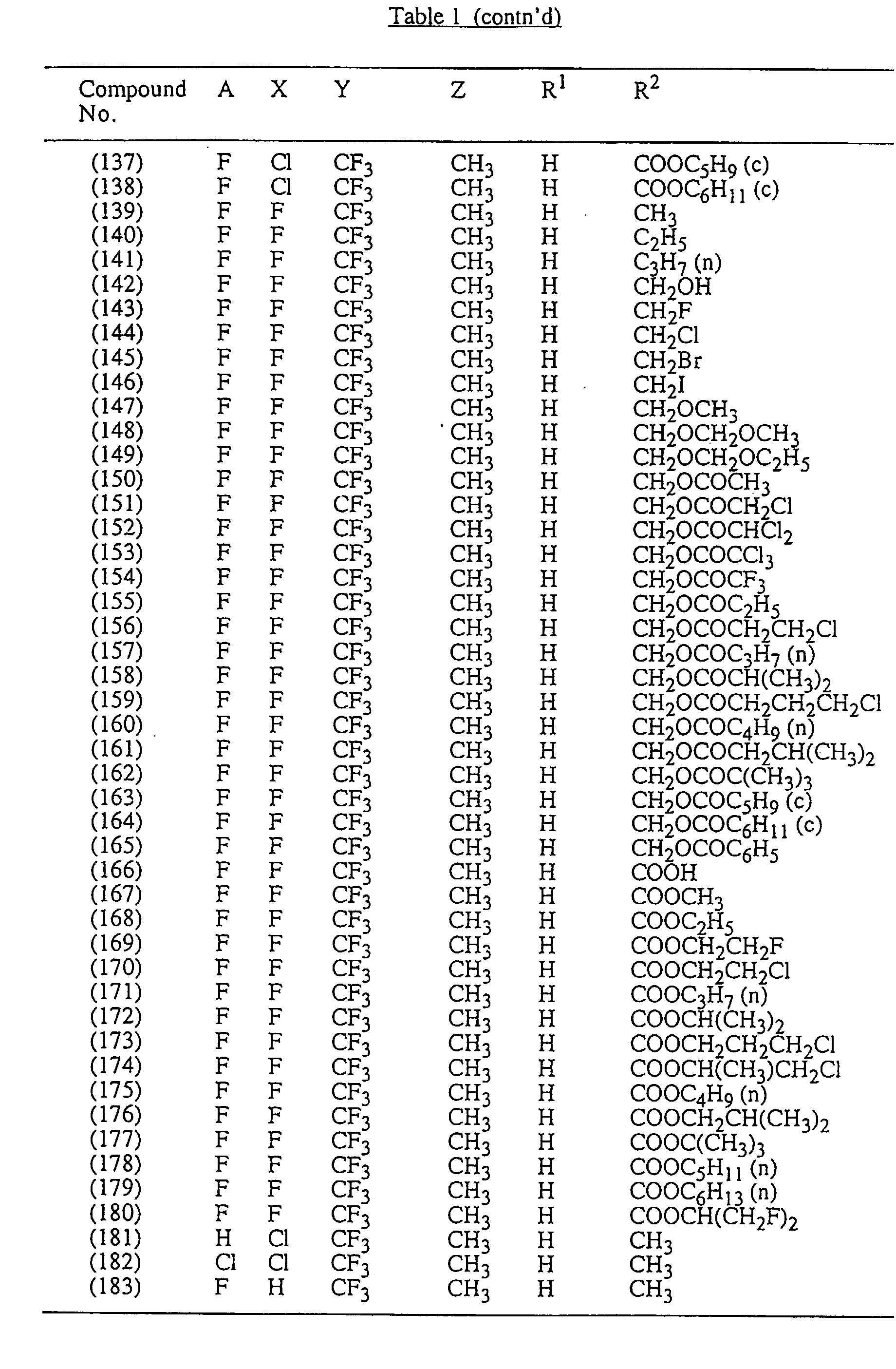

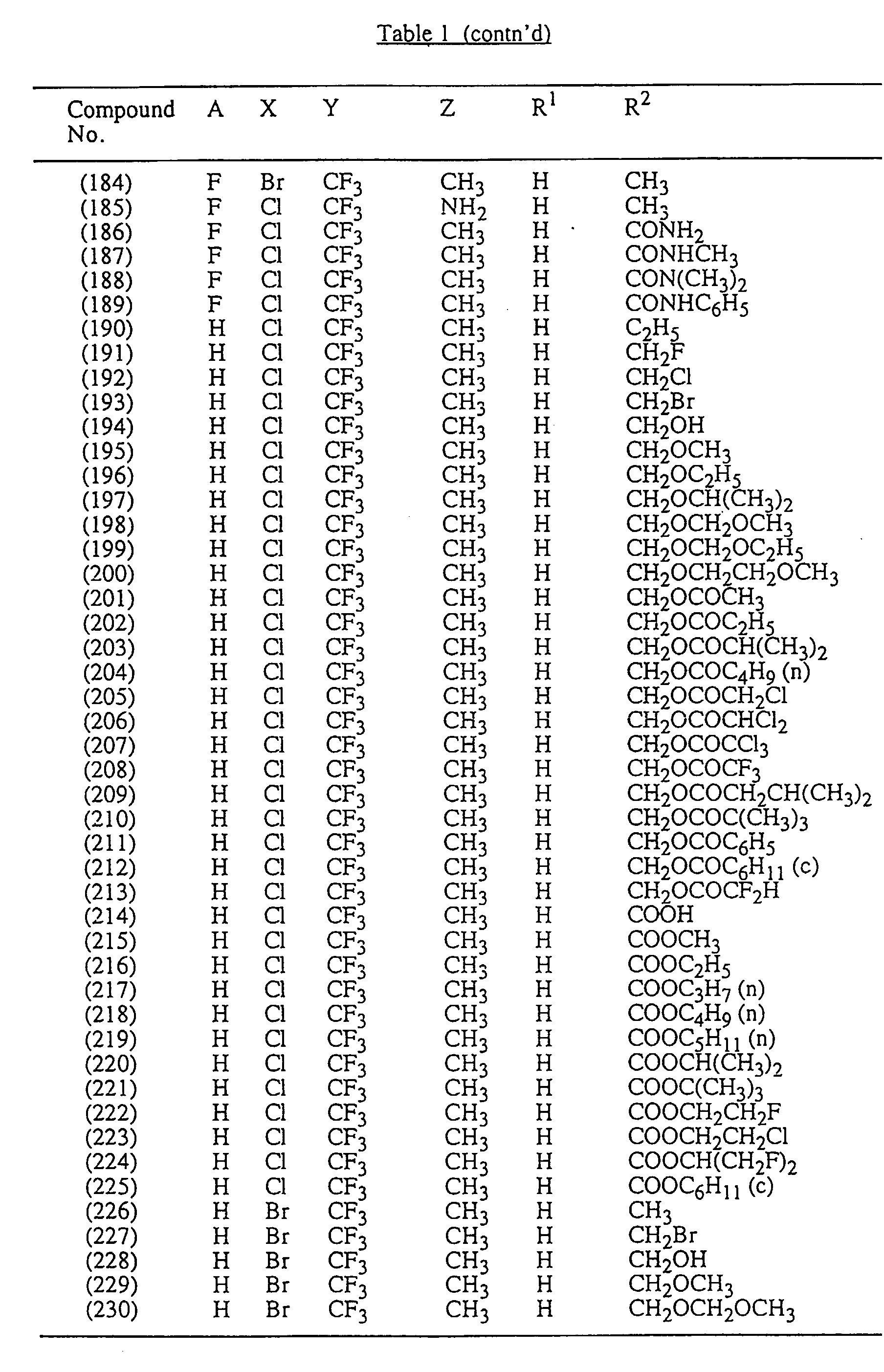

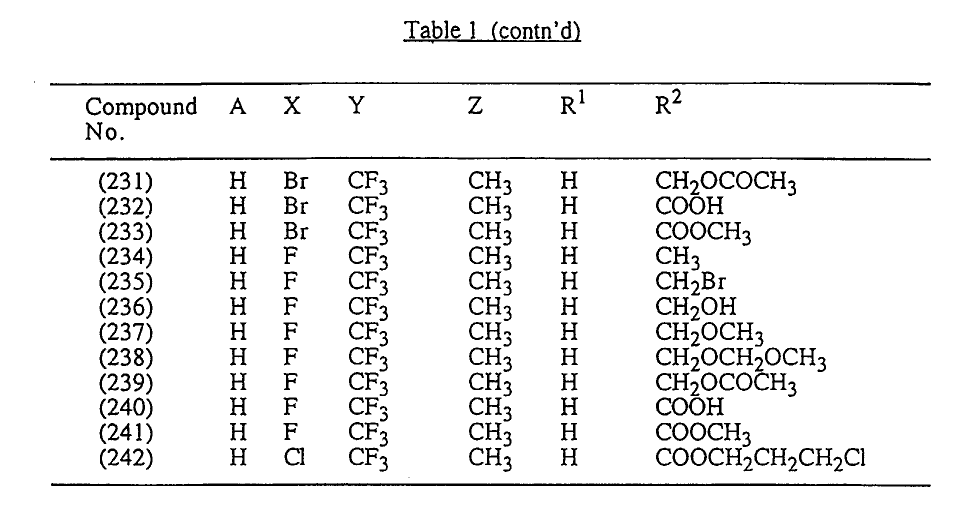

The compounds (I) of the present invention have excellent herbicidal activity and some of them exhibit excellent selectivity between crop plants and weeds. That is, the compounds (I) of the present invention have herbicidal activity against various unfavorable weeds as recited below under the foliar treatment or soil treatment on upland fields.

### Polygonaceae:

wild buckwheat (Polygonum convolvulus), pale smartweed (Polygonum lapathifolium), Pennsylvania smartweed (Polygonum pensylvanicum), ladysthumb (Polygonum persicaria), curly dock (Rumex crispus), broadleaf dock (Rumex obtusifolius)

### Portulacaceae:

common purslane (Portulaca oleracea)

### Caryophyllaceae:

common chickweed (Stellaria media)

### Chenopodiaceae:

common lambsquarters (Chenopodium album), kochia (Kochia scoparia)

### Amaranthaceae:

redroot pigweed (Amaranthus retroflexus), smooth pigweed (Amaranthus hybridus)

### Crusiferae:

wild radish (Raphanus raphanistrum), wild mustard (Brassica kaber), shepherdspurse (Capsella bursa-pastoris)

### Leguminosae:

hemp sesbania (Sesbania exaltata), sicklepod (Cassia obtusifolia), Florida beggarweed (Desmodium tortuosum), white clover (Trifolium repens)

### Malvaceae:

velvetleaf (Abutilon theophrasti), prickly sida (Sida spinosa)

### Violaceae:

field pansy (Viola arvensis), wild pansy (Viola tricolor)

### Rubiaceae:

catchweed bedstraw (cleavers) (Galium aparine)

### Convolvulaceae:

ivyleaf morningglory (Ipomoea hederacea), tall morningglory (Ipomoea purpurea), entireleaf morningglory (Ipomoea hederacea var. integriuscula), pitted morningglory (Ipomoea lacunosa), field bindweed (Convolvulus arvensis)

### Labiatae:

red deadnettle (Lamium purpureum), henbit (Lamium amplexicaule)

### Solanaceae:

jimsonweed (Datura stramonium), black nightshade (Solanum nigrum)

### Scrophulariaceae:

birdseye speedwell (Veronica persica), ivyleaf speedwell (Veronica hederaefolia)

### Compositae:

common cocklebur (Xanthium pensylvanicum), common sunflower (Helianthus annuus), scentless chamomile (Matricaria inodora), corn marigold (Chrysanthemum segetum), pineappleweed (Matricaria matricanioides), common ragweed (Ambrosia artemisiifolia), giant ragweed (Ambrosia trifida), horseweed (Erigeron canadensis)

### Boraginaceae:

field forget-me-not (Myosotis arvensis)

### Asclepiadaceae:

common milkweed (Asclepias syriaca)

### Euphorbiaceae:

sun spurge (Euphorbia helioscopia), spotted spurge (Euphorbia maculata)

### Gramineae:

barnyardgrass (Echinochloa crus-galli), green foxtail (Setaria viridis), giant foxtail (Setaria faberi), large crabgrass (Digitaria sanguinalis), goosegrass (Eleusine indica), annual bluegrass (Poa annua), blackgrass (Alopecurus myosuroides), wild oat (Avena fatua), Johnsongrass (Sorghum halepense), quackgrass (Agropyron repens), downy brome (Bromus tectorum), bermudagrass (Cynodon dactylon), fall panicum (Panicum dichotomiflorum), Texas panicum (Panicum texanum), shattercane (Sorghum vulgare)

### Commelinaceae:

common dayflower (Commelina communis)

### Equisetaceae:

field horsetail (Equisetum arvense)

### Cyperaceae:

rice flatsedge (Cyperus iria), purple nutsedge (Cyperus rotundus), yellow nutsedge (Cyprerus esculentus)

Further, some of the compounds (I) of the present invention have no problematic phytotoxicity on main crops such as corn (Zea mays), wheat (Triticum aestivum), barley (Hordeum vulgare), rice (Oryza sativa), sorghum (Sorghum bicolor), soybean (Glycine max), cotton (Gossypium spp.), sugar beet (Beta vulgaris), peanut (Arachis hypoagea), sunflower (Helianthus annuus) and canola (Brassica napus); and garden crops such as flowers and ornamental plants, and vegetables. In particular, the compounds (I) wherein R² is carboxyl or alkoxycarbonyl have excellent selectivity between soybean and weeds such as tall morningglory, barnyardgrass and Johnsongrass under the soil treatment on upland fields.

In addition, the compounds (I) of the present invention can be very effective against unfavorable weeds in the no-till cultivation. Further, some of them exhibit no problematic phytotoxicity on crop plants such as soybean, corn and wheat.

The compounds (I) of the present invention also have herbicidal activity against various unfavorable weeds as recited below under the flooding treatment on paddy rice fields.

### Gramineae:

barnyardgrass (Echinochloa oryzicola)

### Scrophulariaceae:

common falsepimpernel (Lindernia procumbens)

### Lythraceae:

Rotala indica, Ammannia multiflora

### Elatinaceae:

Elatine triandra

### Cyperaceae:

smallflower umbellaplant (Cyperus difformis), hardstem bulrush (Scirpus juncoides), needle spikerush (Eleocharis acicularis), Cyperus serotinus, Eleocharis kuroauwai

### Pontederiaceae:

Monochoria vaginalis

### Alismataceae:

Satgittaria pygmaea, Sagittaria trifolia, Alisma canaliculatum

### Potamogetonaceae:

roundleaf pondweed (Potamogeton distinctus)

### Umbelliferae:

Oenanthe javanica

Further, some of the compounds (I) of the present invention also have no problematic phytotoxicity on transplanted paddy rice or directly-sown paddy rice.

Further, the compounds (I) of the present invention can be very effective against various unfavorable weeds in orchards, vineyard, plantation, grasslands, lawns or forests, or waterways, canals or other non-cultivated lands.

When the compound (I) of the present invention is used as an active ingredient of herbicides, it is usually formulated with solid or liquid carriers or diluents as well as surfactants and other auxiliary agents into conventional formulations such as emulsifiable concentrates, wettable powders, flowables, granules, concentrated emulsion, water-dispersible granules and solutions.

These formulations contain the compound (I) of the present invention as an active ingredient at a content within the range of 0.003% to 80% by weight, preferably 0.01% to 70% by weight, based on the total weight of each of the formulations.

Examples of the solid carrier or diluent are fine powders or granules of mineral matters such as kaolin clay, attapulgite clay, bentonite, terra alba, pyrophyllite, talc, diatomaceous earth and calcite; organic substances such as walnut shell powder; water-soluble organic substances such as urea; inorganic salts such as ammonium sulfate; and synthetic hydrous silica. As the liquid carrier or diluent, there can be exemplified aromatic hydrocarbons such as alkylbenzenes (e.g., xylene), methylnaphthalene and phenylxylylethane; alcohols such as isopropanol, ethylene glycol and 2-ethoxyethanol; esters such as phthalic acid dialkyl esters; ketones such as acetone, cyclohexanone and isophorone; mineral oils such as machine oil; vegetable oils such as soybean oil and cotton seed oil; dimethylsulfoxide, N,N-dimethylformamide, acetonitrile, N-methylpyrrolidone, water and the like.

Examples of the surfactant used for emulsification, dispersing or spreading are those of anionic type, such as alkylsulfates, alkylsulfonates, alkylarylsulfonates, dialkylsulfosuccinates and phosphates of polyoxyethylene alkylaryl ether; and those of nonionic type, such as polyoxyethylene alkyl ethers, polyoxyethylene alkylaryl ethers, polyoxyethylene polyoxypropylene block copolymers, sorbitan fatty acid esters and polyoxyethylene sorbitan fatty acid esters.

Examples of the auxiliary agent used for formulation are ligninsulfonates, alginates, polyvinyl alcohol, gum arabic, carboxymethyl cellulose (CMC) and isopropyl acid phosphate (PAP).

The compound (I) of the present invention is usually formulated in any suitable formulation and used pre-emergence or post-emergence control of unfavorable weeds in upland fields and paddy fields. The soil treatment includes soil surface treatment and soil incorporation. The foliar treatment includes application over the plants and directed application to the weeds to keep any chemical off the crop foliage.

Further, the compound (I) of the present invention may be used together with other herbicide to enhance its herbicidal activity. Moreover, it may also be used in admixture with insecticides, acaricides, nematocides, fungicides, plant growth regulators, fertilizers, soil improver and the like.

When the compound (I) of the present invention is used as an active ingredient of herbicides, the dosage thereof is usually in the range of from 0.1 to 8000 grams, preferably from 1 to 2000 grams, per hectare, although it may vary depending upon the prevailing weather conditions, formulation type employed, application timing, type of application, soil involved, crop and weed species, and the like. A designated amount of the compound (I) formulated in the form of an emulsifiable concentrate, wettable powder, flowable, concentrated emulsion, water-dispersible granule, solution or the like, may usually be employed by diluting it with water at a volume of about 10 to 1000 liters per hectare, if necessary, with addition of an adjuvant such as a spreading agent. The compound (I) formulated in the form of a granule or some kinds of flowables or solutions may usually be applied without any dilution.

Examples of the adjuvant include, in addition to the surfactants as described above, polyoxyethylene resin acids (esters), ligninsulfonates, abietates, dinaphthylmethanedisulfonates, crop oil concentrates and crop oils such as soybean oil, corn oil, cotton seed oil and sunflower oil.

The compound (I) of the present invention can also be used as an active ingredient of harvesting aids such as defoliants and desiccating agents for cotton and desiccating agents for potato (Solanum tuberosum). In that case, the compound (I) is usually formulated in the same manner as the case where it is used as an active ingredient of herbicides, and used alone or in admixture with other harvesting aids.

The present invention will be further illustrated by way of the following production examples, formulation examples and test examples.

The compounds of the present invention are designated by the corresponding numbers as shown in Table 1.

### Production Example 1: Preparation of Compound (1)

In 5 ml of xylene, 0.5 g of 1-[4-chloro-6-fluoro-3-hydroxy-2-(2-methyl-2-propenyl)phenyl]-3-methyl-4-trifluoromethyl-1,2,3,6-tetrahydropyrimidine-2,6-dione and 50 mg of p-toluenesulfonic acid monohydrate were dissolved, and the solution was refluxed for 5 hours. After completion of the reaction, the reaction solution was poured into water, and the mixture was extracted with 100 ml of diethyl ether. The extract was washed with water, dried and concentrated. The residue was purified by silica gel chromatography (eluent; hexane : ethyl acetate = 2 : 1), which afforded 0.1 g of compound (1).

¹H-NMR δ (ppm) [60 MHz, CDCl₃]: 1.55 (6H, s), 2.9-3.1 (2H, m), 3.61 (3H, s), 6.42 (1H, s), 7.18 (1H, d, J = 10 Hz).

### Production Example 2: Preparation of Compound (4)

In 5 ml of chloroform, 0.4 g of 1-[4-chloro-6-fluoro-3-hydroxy-2-(2-methyl-2-propenyl)phenyl]-3-methyl-4-trifluoromethyl-1,2,3,6-tetrahydropyrimidine-2,6-dione was dissolved, to which a suspension containing 0.26 g of m-chloroperbenzoic acid in 5 ml of chloroform was added dropwise at room temperature, and the reaction was effected under reflux conditions for 3 hours. After completion of the reaction, the reaction mixture was cooled to room temperature, to which a saturated aqueous solution of sodium thiosulfate was added, and the mixture was stirred and extracted with chloroform. The extract was washed with an aqueous solution of sodium hydrogencarbonate, dried and concentrated. The residue was purified by silica gel chromatography (eluent; hexane : ethyl acetate = 2 : 1), which afforded 0.17 g of compound (4).

¹H-NMR δ (ppm) [60 MHz, CDCl₃]: 1.47 (3H, s), 2.3-2.6 (1H, br), 2.8-3.3 (2H, m), 3.56 (3H, s), 3.64 (2H, s), 6.39 (1H, s), 7.11 (1H, d, J = 10 Hz).

### Production Example 3: Preparation of Compound (11)

In 5 ml of pyridine, 0.5 g of compound (4) was dissolved, to which 50 mg of 4-dimethylaminopyridine was added. While ice cooling, 0.2 g of acetic anhydride was added, and the reaction was effected at room temperature for 3 hours. After completion of the reaction, the reaction mixture was extracted with diethyl ether. The extract was washed with diluted hydrochloric acid, dried and concentrated. The residue was purified by silica gel chromatography (eluent; hexane : ethyl acetate = 2 : 1), which afforded 0.2 g of compound (11).

¹H-NMR δ (ppm) [60 MHz, CDCl₃]: 1.29 (3H, s), 1.89 (3H, s), 2.7-2.9 (2H, m), 3.33 (3H, s), 4.00 (2H, s), 6.12 (1H, s), 6.88 (1H, d, J = 10 Hz).

### Production Example 4: Preparation of Compound (15)

In the same manner as described in Production Example 3, except that 0.17 g of propionic anhydride was used in place of acetic anhydride, 0.19 g of compound (15) was obtained.

¹H-NMR δ (ppm) [60 MHz, CDCl₃]: 1.03 (3H, t, J = 8 Hz), 1.51 (3H, s), 2.30 (2H, q, J = 8 Hz), 2.9-3.1 (2H, m), 3.52 (3H, s), 4.19 (2H, s), 6.30 (1H, s), 7.03 (1H, d, J = 10 Hz).

### Production Example 5: Preparation of Compound (23)

In 5 ml of acetone, 0.5 g of compound (4) was dissolved, to which 2 ml of Jones reagent was slowly added dropwise while ice cooling, followed by stirring for 4 hours. After completion of the reaction, the reaction mixture was poured into water, and the mixture was extracted with ethyl acetate. The extract was washed with water, dried and concentrated, which afforded 0.45 g of compound (23).

¹H-NMR δ (ppm) [60 MHz, CDCl₃]: 1.75 (3H, s), 2.9-3.3 (2H, m), 3.53 (3H, s), 6.29 (1H, s), 7.05 (1H, d, J = 10 Hz).

### Production Example 6: Preparation of Compound (24)

In 5 ml of methanol, 0.45 g of compound (23) was dissolved, to which 50 mg of p-toluenesulfonic acid monohydrate was added, and the reaction was effected under reflux conditions for 3 hours. After completion of the reaction, the reaction mixture was extracted with diethyl ether. The extract was washed with water, dried and concentrated. The residue was purified by silica gel chromatography (eluent; hexane : ethyl acetate = 4 : 1), which afforded 0.18 g of compound (24).

¹H-NMR δ (ppm) [60 MHz, CDCl₃]: 1.74 (3H, s), 3.1-3.5 (2H, m), 3.53 (3H, s), 3.76 (3H, s), 6.27 (1H, s), 7.03 (1H, d, J = 10 Hz).

### Production Example 7: Preparation of Compound (25)

In the same manner as described in Production Example 6, except that 0.5 g of compound (23) and 5 ml of ethanol were used, 0.26 g of compound (25) was obtained.

¹H-NMR δ (ppm) [60 MHz, CDCl₃]: 1.24 (3H, t, J = 7 Hz), 1.70 (3H, s), 3.1-3.5 (2H, m), 3.52 (3H, s), 4.22 (2H, q, J = 7 Hz), 6.31 (1H, s), 7.09 (1H, d, J = 10 Hz).

### Production Example 8: Preparation of Compound (32)

In the same manner as described in Production Example 6, except that 0.5 g of compound (23) and 5 ml of 1-butanol were used, 0.13 g of compound (32) was obtained.

¹H-NMR δ (ppm) [60 MHz, CDCl₃]: 0.91 (3H, t, J = 6 Hz), 1.1-1.9 (4H, m), 1.73 (3H, s), 3.1-3.5 (2H, m), 3.55 (3H, s), 4.17 (2H, t, J = 6 Hz), 6.31 (1H, s), 7.08 (1H, d, J = 10 Hz).

### Production Example 9: Preparation of Compound (38)

In the same manner as described in Production Example 6, except that 0.5 g of compound (23) and 1 ml of propargyl alcohol were used, 0.16 g of compound (38) was obtained.

¹H-NMR δ (ppm) [60 MHz, CDCl₃]: 1.74 (3H, s), 2.49 (1H, t, J = 2 Hz), 3.1-3.5 (2H, m), 3.50 (3H, s), 4.71 (2H, d, J = 2 Hz), 6.26 (1H, s), 7.04 (1H, d, J = 10 Hz).

### Production Example 10: Preparation of Compound (39)

In the same manner as described in Production Example 3, except that 0.5 g of compound (4) and 0.17 g of benzoyl chloride were used, 0.18 g of compound (39) was obtained.

m.p., 157-158°C;

¹H-NMR δ (ppm) [60 MHz, CDCl₃]: 1.64 (3H, s), 3.1-3.3 (2H, m), 3.54 (3H, s), 4.3-4.5 (2H, m), 6.35 (1H, s), 7.10 (1H, d, J = 10 Hz), 7.4-7.7 (3H, m), 7.8 - 8.1 (2H, m).

### Production Example 11: Preparation of Compound (49)

In chloroform, 6.0 g of 1-[4-chloro-3-hydroxy-2-(2-methyl-2-propenyl)-phenyl]-3-methyl-4-trifluoromethyl-1,2,3,6-tetrahydropyrimidine-2,6-dione was dissolved, to which 7.1 g of m-chloroperbenzoic acid was added, and the mixture was refluxed for 2 hours. After completion of the reaction, the reaction mixture was cooled to room temperature, to which an aqueous solution of sodium hydrogensulfite was added, and the mixture was extracted with chloroform. The organic layer was washed with aqueous solution potassium carbonate and water, dried and concentrated, which afforded 5.6 g of compound (49).

¹H-NMR δ (ppm) [60 MHz, CDCl₃]: 1.48 (3H, s), 2.05-2.49 (1H, br), 2.87 (1H, s, br), 3.06 (1H, s, br), 3.54 (3H, s), 3.64 (2H, s, br), 6.29 (1H, s), 6.59 (1H, d, J = 8 Hz), 7.22 (1H, s, J = 8 Hz).

### Production Example 12: Preparation of Compound (50)

In 8 ml of N,N-dimethylformamide, 0.3 g of compound (49) was dissolved, to which 0.5 ml of methyl iodide and 0.05 g of sodium hydride (60% in oil) were added at 5°C, and the mixture was warmed to room temperature and stirred for 6 hours. After completion of the reaction, the reaction mixture was poured into water, and the mixture was extracted with ethyl acetate. The extract was washed with water, dried and concentrated. The residue was purified by preparative thin layer chromatography, which afforded 0.12 g of compound (50).

¹H-NMR δ (ppm) [300 MHz, CDCl₃]: 1.52 (3 x ½H, s), 1.53 (3 x ½H, s), 2.78 (½H, d, J = 16 Hz), 2.80 (½H, d, J = 16 Hz), 3.10 (½H, d, J = 16 Hz), 3.13 (½H, d, J = 16 Hz), 3.40 (3 x ½H, s), 3.41 (3 x ½H, s), 3.49 (2H, m), 3.55 (3H, s), 6.33 (½H, s), 6.34 (½H, s), 6.64 (1H, d, J = 8.5 Hz), 7.24 (1H, d, J = 8.5 Hz).

### Production Example 13: Preparation of Compound (53)

In 10 ml of chloroform, 0.3 g of compound (49) was dissolved, to which 0.5 ml of diisopropylethylamine and 0.15 g of chloromethyl methyl ether were added, and the mixture was stirred at room temperature for 17 hours. After completion of the reaction, the reaction mixture was poured into water, and the mixture was extracted with ethyl acetate. The organic layer was dried and concentrated. The residue was purified by preparative thin layer chromatography, which afforded 0.23 g of compound (53).

¹H-NMR δ (ppm) [300 MHz, CDCl₃]: 1.54 (3H, s), 2.82 (½H, d, J = 16 Hz), 2.83 (½H, d, J = 16 Hz), 3.15 (½H, d, J = 16 Hz), 3.16 (½H, d, J = 16 Hz), 3.347 (3 x ½H, s), 3.355 (3 x ½H, s), 3.55 (3H, s), 3.63 (2H, m), 4.65 (2H, m), 6.34 (1H, s), 6.64 (1H, d, J = 8.5 Hz), 7.24 (1H, d, J = 8.5 Hz).

### Production Example 14: Preparation of Compound (56)

To 0.2 g of compound (49), 1.5 ml of pyridine and 1.5 ml of acetic anhydride were added, and the mixture was stirred at room temperature for 15 hours. After completion of the reaction, the reaction mixture was concentrated. The residue was purified by preparative thin layer chromatography, which afforded 0.18 g of compound (56).

¹H-NMR δ (ppm) [250 MHz, CDCl₃]: 1.58 (3H, s), 2.07 (3H, s), 2.92 (½H, d, J = 16 Hz), 2.93 (½H, d, J = 16 Hz), 3.12 (1H, d, J = 16 Hz), 3.59 (3H, s), 4.22 (1H, d, J = 12 Hz), 4.30 (½H, d, J = 12 Hz), 4.31 (½H, d, J = 12 Hz), 6.38 (1H, s), 6.69 (1H, d, J = 8.5 Hz), 7.30 (1H, d, J = 8.5 Hz).

### Production Example 15: Preparation of Compound (69)

In 40 ml of acetone, 3.0 g of compound (49) was dissolved, to which Jones reagent was added at room temperature until the starting material disappeared. After completion of the reaction, 2-propanol was added to the reaction mixture, which was stirred for 1 hour. The reaction mixture was poured into water, and the mixture was extracted with diethyl ether. The organic layer was washed with water, dried and concentrated, which afforded 2.72 g of compound (69).

¹H-NMR δ (ppm) [60 MHz, CDCl₃]: 1.70 (3H, s), 3.11 (1H, s, br), 3.38 (1H, s, br), 3.49 (3H, s, br), 6.26 (1H, s), 6.61 (1H, d, J = 8 Hz), 7.19 (1H, d, J = 8 Hz).

### Production Example 16: Preparation of Compound (70)

In 30 ml of methanol, 0.5 g of compound (69) was dissolved, to which 0.1 ml of sulfuric acid was added, and the mixture was refluxed for 3 hours. After completion of the reaction, the reaction mixture was poured into water, and the mixture was extracted with diethyl ether. The extract was washed with water, dried and concentrated. The residue was purified by preparative thin layer chromatography, which afforded 0.24 g of compound (70).

¹H-NMR δ (ppm) [60 MHz, CDCl₃]: 1.74 (3H, s), 3.15 (1H, s, br), 3.42 (1H, s, br), 3.52 (3H, s), 3.77 (3H, s), 6.29 (1H, s), 6.64 (1H, d, J = 8 Hz), 7.23 (1H, d, J = 8 Hz).

### Production Example 17: Preparation of Compound (72)

In the same manner as described in Production Example 16, except that 30 ml of 1-propanol was used in place of methanol, 0.3 g of compound (72) was obtained.

¹H-NMR δ (ppm) [60 MHz, CDCl₃]: 0.90 (3H, t, J = 7 Hz), 1.39-1.85 (2H, m), 1.74 (3H, s), 3.16 (1H, s), 3.41 (1H, s), 3.52 (3H, s), 4.11 (2H, t, J = 7 Hz), 6.31 (1H, s), 6.64 (1H, d, J = 8 Hz), 7.24 (1H, d, J = 8 Hz).

### Production Example 18: Preparation of Compound (73)

In the same manner as described in Production Example 16, except that 30 ml of 1-butanol was used in place of methanol, 0.2 g of compound (73) was obtained.

¹H-NMR δ (ppm) [60 MHz, CDCl₃]: 0.90 (3H, t, J = 7 Hz), 1.12-1.92 (4H, m), 1.72 (3H, s), 3.14 (1H, s), 3.40 (1H, s), 3.51 (3H, s), 4.14 (2H, t, J = 7 Hz), 6.29 (1H, s), 6.63 (1H, d, J = 8 Hz), 7.72 (1H, d, J = 8 Hz).

### Production Example 19: Preparation of Compound (75)

In the same manner as described in Production Example 16, except that 30 ml of 2-propanol was used in place of methanol, 0.3 g of compound (75) was obtained.

¹H-NMR δ (ppm) [60 MHz, CDCl₃]: 1.37 (6H, d, J = 7 Hz), 1.73 (3H, s), 3.14 (3H, s), 3.39 (1H, s), 3.52 (1H, s), 5.02 (1H, hp, J = 7 Hz), 6.31 (1H, s), 6.63 (1H, d, J = 8 Hz), 7.24 (1H, d, J = 8 Hz).

### Production Example 20: Preparation of Compound (104)

In 600 ml of chloroform, 58 g of 1-(2-allyl-4-chloro-6-fluoro-3-hydroxy-phenyl)-3-methyl-4-trifluoromethyl-1,2,3,6-tetrahydropyrimidine-2,6-dione was dissolved, to which a suspension containing 38 g of m-chloroperbenzoic acid in 300 ml of chloroform was added dropwise at room temperature, and the mixture was heated under reflux. After 6 hours, the reaction mixture was cooled to room temperature, to which a saturated aqueous solution of sodium thiosulfate was added, and the mixture was stirred. After completion of the reaction, the organic layer was washed with an aqueous solution of sodium hydrogencarbonate, dried and concentrated. The residue was purified by silica gel chromatography (eluent; hexane : ethyl acetate = 3:1), which afforded 43.2 g of compound (104) as colorless crystals.

m.p., 104.1°C;

¹H-NMR δ (ppm) [60 MHz, CDCl₃]: 3.10 (2H, d, J = 6 Hz), 3.56 (3H, s), 3.65-3.85 (2H, m), 5.0-5.2 (1H, m), 6.33 (1H, s), 7.05 (1H, d, J = 10 Hz).

### Production Example 21: Preparation of Compound (105)

In 15 ml of methylene chloride, 0.5 g of compound (104) was dissolved, and the solution was cooled to 5°C in ice water, to which 0.22 g of diethylaminosulfuric trifluoride (DAST) was slowly added dropwise, which was stirred for 2 hours. After completion of the reaction, the reaction mixture was poured into water, and the mixture was extracted with methylene chloride. The extract was washed with an aqueous solution of sodium hydrogencarbonate, dried and concentrated. The residue was purified by silica gel chromatography (eluent; hexane : ethyl acetate = 4 : 1), which afforded 0.26 g of compound (105).

¹H-NMR δ (ppm) [60 MHz, CDCl₃]: 3.13 (1H, d, J = 8 Hz), 3.16 (1H, d, J = 9 Hz), 3.54 (3H, s), 4.59 (2H, dd, J = 4, 47 Hz), 4.8-5.4 (1H, m), 6.33 (1H, s), 7.10 (1H, d, J = 10 Hz).

### Production Example 22: Preparation of Compound (107)

In 15 ml of dichloromethane, 1.30 g of compound (104) and 1.3 g of carbon tetrachloride were dissolved, to which 1.2 g of triphenylphosphine was added at 5°C, and the mixture was stirred at room temperature for 24 hours. After completion of the reaction, the reaction mixture was concentrated. The residue was purified by silica gel chromatography (eluent; hexane : ethyl acetate = 3 : 1), which afforded 1.43 g of compound (107).

¹H-NMR δ (ppm) [60 MHz, CDCl₃]: 3.0-3.3 (2H, m), 3.45-3.55 (2H, m), 3.50 (3H, s), 4.9-5.3 (1H, m), 6.22 (1H, s), 6.96 (1H, d, J = 10 Hz).

### Production Example 23: Preparation of Compound (111)

In 10 ml of pyridine, 0.5 g of compound (104) and 50 mg of 4-dimethylaminopyridine were dissolved, to which 0.15 g of acetic anhydride was added, and the mixture was stirred at room temperature for 1 hours. After completion of the reaction, the reaction mixture was poured into water, and the mixture was extracted twice with 100 ml of diethyl ether. The extract was washed with diluted hydrochloric acid, dried and concentrated. The residue was purified by silica gel chromatography (eluent; hexane : ethyl acetate = 3 : 1), which afforded 0.39 g of compound (111).

¹H-NMR δ (ppm) [60 MHz, CDCl₃]: 2.03 (3H, s), 2.99 (1H, d, J = 7 Hz), 3.12 (1H, d, J = 9 Hz), 3.50 (3H, s), 4.24 (2H, d, J = 5 Hz), 4.9-5.2 (1H, m), 6.25 (1H, s), 6.97 (1H, d, J = 6 Hz).

### Production Example 24: Preparation of Compound (112)

In the same manner as described in Production Example 23, except that 0.5 g of compound (104) and 0.26 g of monochloroacetic anhydride were used as the starting materials, 0.35 g of compound (112) was obtained.

¹H-NMR δ (ppm) [60 MHz, CDCl₃]: 3.00 (1H, d, J = 7 Hz), 3.11 (1H, d, J = 10 Hz), 3.50 (3H, s), 4.01 (2H, s), 4.36 (2H, d, J = 4 Hz), 4.9-5.3 (1H, m), 6.24 (1H, s), 6.98 (1H, d, J = 10 Hz).

### Production Example 25: Preparation of Compound (114)

In the same manner as described in Production Example 23, except that 0.5 g of compound (104) and 0.277 g of trichloroacetyl chloride were used as the starting materials, 0.3 g of compound (114) was obtained.

¹H-NMR δ (ppm) [60 MHz, CDCl₃]: 3.15 (1H, d, J = 7 Hz), 3.28 (1H, d, J = 9 Hz), 3.56 (3H, s), 4.59 (2H, d, J = 4 Hz), 5.05-5.40 (1H, m), 6.54 (1H, s), 7.09 (1H, d, J = 10 Hz).

### Production Example 26: Preparation of Compound (115)

In the same manner as described in Production Example 23, except that 0.5 g of compound (104) and 0.198 g of propionic anhydride were used as the starting materials, 0.34 g of compound (115) was obtained.

¹H-NMR δ (ppm) [60 MHz, CDCl₃]: 1.08 (3H, t, J = 7 Hz), 2.32 (2H, q, J = 7 Hz), 2.98 (1H, d, J = 7 Hz), 3.11 (1H, d, J = 8 Hz), 3.49 (3H, s), 4.26 (2H, d, J = 4 Hz), 5.0-5.1 (1H, m), 6.22 (1H, s), 6.94 (1H, t, J = 10 Hz).

### Production Example 27: Preparation of Compound (116)

In the same manner as described in Production Example 23, except that 0.5 g of compound (104) and 0.24 g of butyric anhydride were used as the starting materials, 0.37 g of compound (116) was obtained.

¹H-NMR δ (ppm) [60 MHz, CDCl₃]: 0.90 (3H, t, J = 6 Hz), 1.61 (2H, tq, J = 6, 6 Hz), 2.30 (2H, t, J = 6 Hz), 3.02 (1H, d, J = 7 Hz), 3.15 (1H, d, J = 9 Hz), 3.51 (3H, s), 4.28 (2H, d, J = 4 Hz), 4.95-5.25 (1H, m), 6.28 (1H, s), 7.00 (1H, d, J = 10 Hz).

### Production Example 28: Preparation of Compound (117)

In the same manner as described in Production Example 23, except that 0.5 g of compound (104) and 0.24 g of isobutyric anhydride were used as the starting materials, 0.31 g of compound (117) was obtained.

¹H-NMR δ (ppm) [60 MHz, CDCl₃]: 1.12 (6H, d, J = 7 Hz), 2.3-2.9 (3H, m), 3.0-3.3 (2H, m), 3.50 (3H, s), 4.26 (2H, d, J = 4 Hz), 4.9-5.3 (1H, m), 6.25 (1H, s), 7.07 (1H, d, J = 10 Hz).

### Production Example 29: Preparation of Compound (120)

In the same manner as described in Production Example 23, except that 0.5 g of compound (104) and 0.18 g of pivaloyl chloride were used as the starting materials, 0.29 g of compound (120) was obtained.

m.p., 145°C;

¹H-NMR δ (ppm) [60 MHz, CDCl₃]: 1.14 (9H, s), 2.8-3.0 (2H, m), 3.56 (3H, s), 4.20-4.40 (2H, m), 5.1-5.3 (1H, m), 6.31 (1H, s), 7.15 (1H, d, J = 10 Hz).

### Production Example 30: Preparation of Compound (121)

In the same manner as described in Production Example 23, except that 0.5 g of compound (104) and 0.2 g of cyclopentanecarbonyl chloride were used as the starting materials, 0.42 g of compound (121) was obtained.

¹H-NMR δ (ppm) [60 MHz, CDCl₃]: 1.5-1.9 (8H, m), 2.6-2.8 (1H, m), 3.04 (1H, d, J = 7 Hz), 3.17 (1H, d, J = 9 Hz), 3.53 (3H, s), 4.31 (2H, d, J = 4 Hz), 4.95-5.30 (1H, m), 6.37 (1H, s), 7.11 (1H, d, J = 10 Hz).

### Production Example 31: Preparation of Compound (122)

In the same manner as described in Production Example 23, except that 0.5 g of compound (104) and 0.22 g of cyclohexanecarbonyl chloride were used as the starting materials, 0.41 g of compound (122) was obtained.

¹H-NMR δ (ppm) [60 MHz, CDCl₃]: 1.15-1.95 (10H, m), 2.2-2.4 (1H, m), 3.05 (1H, d, J = 8 Hz), 3.18 (1H, d, J = 9 Hz), 3.58 (3H, s), 4.32 (2H, d, J = 4 Hz), 5.0-5.3 (1H, m), 6.37 (1H, s), 7.08 (1H, d, J = 10 Hz).

### Production Example 32: Preparation of Compound (123)

In 150 ml of acetone, 20 g of compound (104) was dissolved, to which 40 ml of Jones reagent was slowly added dropwise while ice cooling, and the mixture was stirred for 2 hours. After completion of the reaction, about 20 ml of isopropyl alcohol was added dropwise, which was stirred. The reaction mixture was poured into water, and the mixture was extracted with ethyl acetate. The extract was washed with water, dried and concentrated, which afforded 15.3 g of compound (123).

¹H-NMR δ (ppm) [60 MHz, CDCl₃]: 3.2-3.4 (2H, m), 3.51 (3H, s), 5.1-5.4 (1H, m), 6.30 (1H, s), 7.02 (1H, d, J = 10 Hz).

### Production Example 33: Preparation of compound (124)

In 15 ml of methanol, 0.7 g of compound (123) was dissolved, to which 0.05 g of p-toluenesulfonic acid was added, and the mixture was heated under reflux for 2 hours. After completion of the reaction, the reaction mixture was poured into water, and the mixture was extracted with diethyl ether. The extract was washed with water, dried and concentrated. The residue was purified by silica gel chromatography (eluent; hexane : : ethyl acetate = 3 1) which afforded 0.4 g of compound (124).

¹H-NMR δ (ppm) [60 MHz, CDCl₃]: 3.37 (2H, d, J = 9 Hz), 3.48 (3H, s), 3.72 (3H, s), 5.28 (1H, t, J = 9 Hz), 6.23 (1H, s), 7.01 (1H, d, J = 10 Hz).

### Production Example 34: Preparation of Compound (125)

In the same manner as described in Production Example 33, except that 15 ml of ethanol was used in place of methanol, 0.38 g of compound (125) was obtained.

¹H-NMR δ (ppm) [60 MHz, CDCl₃]: 1.26 (3H, t, J = 7 Hz), 3.33 (2H, d, J = 7 Hz), 3.47 (3H, s), 4.18 (2H, q, J = 7 Hz), 5.24 (1H, t, J = 7 Hz), 6.20 (1H, s), 7.01 (1H, d, J = 10 Hz).

### Production Example 35: Preparation of Compound (126)

In the same manner as described in Production Example 33, except that 15 ml of 2-fluoroethanol was used in place of methanol, 0.28 g of compound (126) was obtained.

¹H-NMR δ (ppm) [60 MHz, CDCl₃]: 3.25-3.45 (2H, m), 3.50 (3H, s), 4.15 (2H, s), 4.79 (2H, dt, J = 21, 4 Hz), 5.33 (1H, dd, J = 7, 9 Hz), 6.28 (1H, s), 7.06 (1H, d, J = 10 Hz).

### Production Example 36: Preparation of Compound (127)

In the same manner as described in Production Example 33, except that 15 ml of 2-chloroethanol was used in place of methanol, 0.32 g of compound (127) was obtained.

¹H-NMR δ (ppm) [60 MHz, CDCl₃]: 3.37 (1H, d, J = 7 Hz), 3.39 (1H, d, J = 8 Hz), 3.50 (3H, s), 3.66 (2H, t, J = 6 Hz), 4.40 (2H, t, J = 6 Hz), 5.31 (1H, dd, J = 7, 8 Hz), 6.26 (1H, s), 7.04 (1H, d, J = 10 Hz).

### Production Example 37: Preparation of Compound (128)

In the same manner as described in Production Example 33, except that 15 ml of n-propyl alcohol was used in place of methanol, 0.44 g of compound (128) was obtained.

¹H-NMR δ (ppm) [60 MHz, CDCl₃]: 1.41 (3H, t, J = 7 Hz), 1.16 (2H, m, J = 7 Hz), 3.36 (2H, d, J = 8 Hz), 3.49 (3H, s), 4.11 (2H, t, J = 7 Hz), 5.29 (1H, t, J = 8 Hz), 6.26 (1H, s), 7.03 (1H, d, J = 10 Hz).

### Production Example 38: Preparation of Compound (129)

In the same manner as described in Production Example 33, except that 15 ml of isopropyl alcohol was used in place of methanol, 0.36 g of compound (129) was obtained.

¹H-NMR δ (ppm) [60 MHz, CDCl₃]: 1.24 (6H, d, J = 6 Hz), 3.28 (1H, d, J = 8 Hz), 3.30 (1H, d, J = 6 Hz), 3.46 (3H, s), 4.98 (1H, m), 5.19 (1H, dd, J = 6, 8 Hz), 6.20 (1H, s), 6.97 (1H, d, J = 10 Hz).

### Production Example 39: Preparation of Compound (131)

In the same manner as described in Production Example 33, except that 15 ml of 3-chloropropanol was used in place of methanol, 0.45 g of compound (131) was obtained.

¹H-NMR δ (ppm) [60 MHz, CDCl₃]: 1.8-2.2 (4H, m), 3.25-3.45 (2H, m), 3.50 (3H, s), 3.70 (2H, t, J = 6 Hz), 4.28 (2H, t, J = 6 Hz), 5.25 (1H, dd, J = 8, 9 Hz), 6.23 (1H, s), 7.00 (1H, d, J = 10 Hz).

### Production Example 40: Preparation of Compound (132)

In the same manner as described in Production Example 33, except that 15 ml of 3-chloro-2-propanol was used in place of methanol, 0.31 g of compound (132) was obtained.

¹H-NMR δ (ppm) [60 MHz, CDCl₃]: 1.39 (3H, d, J = 6 Hz), 3.35-3.60 (2H, m), 3.60 (3H, s), 3.65 (2H, d, J = 6 Hz), 5.15-5.45 (1H, m), 5.45 (1H, dd, J = 8, 9 Hz), 6.44 (1H, s), 7.23 (1H, d, J = 10 Hz).

### Production Example 41: Preparation of Compound (133)

In the same manner as described in Production Example 33, except that 15 ml of 1,3-difluoro-2-propanol was used in place of methanol, 0.27 g of compound (133) was obtained.

¹H-NMR δ (ppm) [60 MHz, CDCl₃]: 3.25-3.45 (2H, m), 3.51 (3H, s), 4.0-4.3 (1H, m), 4.57 (4H, m), 5.1-5.5 (1H, m), 6.29 (1H, s), 7.05 (1H, d, J = 10 Hz).

### Production Example 42: Preparation of Compound (138)

In the same manner as described in Production Example 33, except that 15 ml of cyclohexanol was used in place of methanol, 0.3 g of compound (138) was obtained.

¹H-NMR δ (ppm) [60 MHz, CDCl₃]: 1.1-2.0 (10H, m), 3.34 (1H, d, J = 7 Hz), 3.40 (1H, d, J = 9 Hz), 3.53 (3H, s), 4.7-5.1 (1H, m), 5.27 (1H, dd, J = 7, 9 Hz), 6.31 (1H, s), 7.07 (1H, d, J = 10 Hz).

### Production Example 43: Preparation of Compound (139)

In 5 ml of xylene, 0.20 g of 1-(2-allyl-4,6-difluoro-3-hydroxyphenyl)-3-methyl-4-trifluoromethyl-1,2,3,6-tetrahydropyrimidine-2,6-dione and 10 mg of p-toluenesulfonic acid monohydrate were dissolved, and the solution was refluxed for 7 hours. After completion of the reaction, the reaction mixture was poured into water, and the mixture was extracted with 100 ml of diethyl ether. The organic layer was washed with water, dried and concentrated. The residue was purified by silica gel chromatography (eluent; hexane : ethyl acetate = 2 : 1), which afforded 0.13 g of compound (139).

¹H-NMR δ (ppm) [60 MHz, CDCl₃]: 1.47 (3H, d, J = 6 Hz), 2.8-3.2 (2H, m), 3.49 (3H, s), 4.9-5.2 (1H, m), 6.22 (1H, s), 6.73 (1H, t, J = 10 Hz).

### Production Example 44: Preparation of Compound (142)

In 24 ml of methylene chloride, 1.0 g of 1-(2-allyl-4,6-difluoro-3-hydroxy-phenyl)-3-methyl-4-trifluoromethyl-1,2,3,6-tetrahydropyrimidine-2,6-dione was dissolved, to which a suspension containing 0.71 g of m-chloroperbenzoic acid in 12 ml of methylene chloride was added dropwise at room temperature, and the reaction was effected under reflux conditions for 3 hours. After completion of the reaction, the reaction mixture was cooled to room temperature, to which a saturated aqueous solution of sodium thiosulfate was added, and the mixture was stirred and extracted with chloroform. The extract was washed with an aqueous solution of sodium hydrogencarbonate, dried and concentrated to remove the solvent. The residue was purified by silica gel chromatography (eluent; hexane : ethyl acetate = 7 : 3), which afforded 1.1 g of compound (142) as yellowish white crystals.

m.p., 57.6°C;

¹H-NMR δ (ppm) [60 MHz, CDCl₃]: 3.0 (2H, d, J = 6 Hz), 3.5 (3H, s), 3.65-3.86 (2H, m), 4.81-5.25 (1H, m), 6.27 (1H, s), 6.77 (1H, t, J = 11 Hz).

### Production Example 45: Preparation of Compound (147)

In 3 ml of dimethylformamide, 0.05 g of sodium hydride was suspended, to which a solution containing 0.5 g of compound (142) in 1 ml of dimethylformamide was added, and the mixture was stirred for 10 minutes. Then, 0.4 g of methyl iodide was added thereto, and the mixture was stirred at room temperature for 6 hours. After completion of the reaction, the reaction mixture was extracted with ethyl acetate. The extract was washed with water, dried and concentrated. The residue was purified by silica gel chromatography (eluent; hexane : ethyl acetate = 3 : 1), which afforded 0.25 g of compound (147).

¹H-NMR δ (ppm) [60 MHz, CDCl₃]: 2.9-3.2 (2H, m), 3.40 (3H, s), 3.55 (3H, s), 3.60 (2H, d, J = 5 Hz), 4.9-5.3 (1H, m), 6.31 (1H, s), 6.83 (1H, t, J = 10 Hz).

### Production Example 46: Preparation of Compound (150)

In 5 ml of pyridine, 0.3 g of compound (142) was dissolved, to which 50 mg of 4-dimethylaminopyridine was added. While ice cooling, 0.12 g of acetic anhydride was added thereto, and the reaction was effected at room temperature for 3 hours. After completion of the reaction, the reaction mixture was extracted with diethyl ether. The extract was washed with diluted hydrochloric acid, dried and evaporated to remove the solvent. The residue was purified by silica gel chromatography (eluent; hexane : ethyl acetate = 7 : 3), which afforded 0.25 g of compound (150) as white crystals.

m.p., 47.2°C;

¹H-NMR δ (ppm) [60 MHz, CDCl₃]: 2.11 (3H, s), 3.16 (2H, t, J = 10 Hz), 3.62 (3H, s), 4.43 (2H, d, J = 4 Hz), 4.92-5.41 (1H, m), 6.44 (1H, s), 6.92 (1H, t, J = 11 Hz).

### Production Example 47: Preparation of Compound (152)

In 1.1 ml of pyridine, 0.5 g of compound (142) and 50 mg of 4-dimethylaminopyridine were dissolved, to which 0.20 g of dichloroacetyl chloride was added while ice cooling, and the reaction was effected at room temperature for 6 hours. After completion of the reaction, the reaction mixture was extracted with ethyl acetate. The extract was washed with diluted hydrochloric acid, dried and evaporated to remove the solvent. The residue was purified by silica gel chromatography (eluent; hexane : ethyl acetate = 7 : 3), which afforded 0.23 g of compound (152).

¹H-NMR δ (ppm) [60 MHz, CDCl₃]: 2.93-3.34 (2H, m), 3.51 (3H, s), 4.42 (2H, d, J = 5 Hz), 4.92-5.37 (1H, m), 5.91 (1H, s), 6.23 (1H, s), 6.77 (1H, t, J = 10 Hz).

### Production Example 48: Preparation of Compound (153)

In the same manner as described in Production Example 47, except that 0.5 g of compound (142) and 0.24 g of trichloroacetyl chloride were used as the starting materials, 0.3 g of compound (153) was obtained.

¹H-NMR δ (ppm) [60 MHz, CDCl₃]: 3.07-3.43 (2H, m), 3.67 (3H, s), 4.67 (2H, d, J = 5 Hz), 5.13-5.56 (1H, m), 6.48 (1H, s), 7.01 (1H, t, J = 10 Hz).

### Production Example 49: Preparation of Compound (154)

In 16 ml of tetrahydrofuran, 0.5 g of compound (142) and 0.19 g of 4-dimethylaminopyridine were dissolved, to which 0.57 g of trifluoroacetic anhydride was added while ice cooling, and the reaction was effected at room temperature for 3 hours. After completion of the reaction, the reaction mixture was extracted with diethyl ether. The extract was washed with water and then brine, dried and evaporated to remove the solvent. The residue was purified by silica gel chromatography (eluent; hexane : ethyl acetate = 7 : 3), which afforded 0.3 g of compound (154).

¹H-NMR δ (ppm) [60 MHz, CDCl₃]: 2.68-3.01 (2H, m), 3.81 (3H, s), 4.85 (2H, d, J = 5 Hz), 5.3-5.85 (1H, m), 6.70 (1H, s), 7.25 (1H, t, J = 10 Hz).

### Production Example 50: Preparation of Compound (155)

In the same manner as described in Production Example 49, except that 0.5 g of compound (142) and 0.17 g of propionic anhydride were used as the starting materials, 0.48 g of compound (155) was obtained.

¹H-NMR δ (ppm) [60 MHz, CDCl₃]: 1.05 (3H, t, J = 7 Hz), 2.31 (2H, q, J = 7 Hz), 3.0 (2H, t, J = 7 Hz), 3.50 (3H, s), 4.40 (2H, d, J = 6 Hz), 4.87-5.27 (1H, m), 6.23 (1H, s), 6.74 (1H, t, J = 11 Hz).

### Production Example 51: Preparation of Compound (157)

In the same manner as described in Production Example 49, except that 0.5 g of compound (142) and 0.21 g of butyric anhydride were used as the starting materials, 0.6 g of compound (157) was obtained.

¹H-NMR δ (ppm) [60 MHz, CDCl₃]: 0.92 (3H, t, J = 7 Hz), 1.42-2.05 (2H, m), 2.35 (2H, t, J = 8 Hz), 3.10 (2H, t, J = 8 Hz), 3.58 (3H, s), 4.35 (2H, d, J = 5 Hz), 5.00-5.52 (1H, m), 6.42 (1H, s), 6.98 (1H, t, J = 11 Hz).

### Production Example 52: Preparation of Compound (158)

In the same manner as described in Production Example 47, except that 0.5 g of compound (142) and 0.17 g of isobutyryl chloride were used as the starting materials, 0.21 g of compound (158) was obtained.

¹H-NMR δ (ppm) [60 MHz, CDCl₃]: 1.08 (3H, s), 1.19 (3H, s), 2.24 - 2.81 (1H, m), 3.09 (2H, t, J = 8 Hz), 3.57 (3H, s), 4.30 (2H, d, J = 4 Hz), 4.94-5.37 (1H, m), 6.32 (1H, s), 6.87 (1H, t, J = 10 Hz).

### Production Example 53: Preparation of Compound (165)

In the same manner as described in Production Example 47, except that 0.5 g of compound (142) and 0.19 g of benzoyl chloride were used as the starting materials, 0.28 g of compound (165) was obtained.

¹H-NMR δ (ppm) [60 MHz, CDCl₃]: 3.09-3.30 (2H, m), 3.98 (3H, s), 4.41-4.54 (2H, m), 5.02-5.43 (1H, m), 6.38 (1H, s), 6.81 (1H, t, J = 10 Hz), 7.21 - 8.10 (5H, m).

### Production Example 54: Preparation of Compound (166)

In 30 ml of acetone, 4 g of compound (142) was dissolved, to which 10 ml of Jones reagent was slowly added dropwise while ice cooling, and the mixture was stirred for 2 hours. After completion of the reaction, about 5 ml of isopropyl alcohol was added dropwise thereto, and the mixture was stirred. The reaction mixture was poured into water, and the mixture was extracted with ethyl acetate. The extract was washed with water, dried and evaporated to remove the solvent, which afforded 3.95 g of compound (166).

¹H-NMR δ (ppm) [60 MHz, CDCl₃]: 2.9-3.1 (2H, m), 3.49 (3H, s), 4.7-5.2 (1H, m), 6.23 (1H, s), 6.73 (1H, t, J = 10 Hz).

### Production Example 55: Preparation of Compound (167)

In 0.42 g of methanol, 0.5 g of compound (166) was dissolved, to which 50 mg of p-toluenesulfonic acid monohydrate was added, and the reaction was effected under reflux conditions for 4 hours. After completion of the reaction, the reaction mixture was extracted with diethyl ether. The extract was washed with water, dried and evaporated to remove the solvent. The residue was purified by silica gel chromatography (eluent; hexane : : 1), ethyl acetate = 4 which afforded 0.24 g of compound (167).

¹H-NMR δ (ppm) [60 MHz, CDCl₃]: 3.29-3.47 (2H, m), 3.52 (3H, s), 3.75 (3H, s), 5.30 (1H, t, J = 9 Hz), 6.25 (1H, s), 6.82 (1H, t, J = 10 Hz).

### Production Example 56: Preparation of Compound (168)

In the same manner as described in Production Example 55, except that 0.61 g of ethanol was used in place of methanol, 0.13 g of compound (168) was obtained.

¹H-NMR δ (ppm) [60 MHz, CDCl₃]: 1.27 (3H, t, J = 7 Hz), 3.27-3.45 (2H, m), 3.53 (3H, s), 4.23 (2H, q, J = 7 Hz), 5.28 (1H, t, J = 9 Hz), 6.26 (1H, s), 6.85 (1H, t, J = 10 Hz).

### Production Example 57: Preparation of Compound (171)

In the same manner as described in Production Example 55, except that 0.79 g of 1-propanol was used in place of methanol, 0.29 g of compound (171) was obtained.

¹H-NMR δ (ppm) [60 MHz, CDCl₃]: 0.94 (3H, t, J = 8 Hz), 1.25-1.85 (2H, m), 3.37-3.43 (2H, m), 3.53 (3H, s), 4.23 (2H, t, J = 7 Hz), 5.28 (1H, t, J = 9 Hz), 6.27 (1H, s), 6.85 (1H, t, J = 10 Hz).

### Production Example 58: Preparation of Compound (172)

In the same manner as described in Production Example 55, except that 0.79 g of 2-propanol was used in place of methanol, 0.12 g of compound (172) was obtained.

¹H-NMR δ (ppm) [60 MHz, CDCl₃]: 1.28 (6H, d, J=6 Hz), 3.27-3.44 (2H, m), 3.53 (3H, s), 4.90-5.50 (2H, m), 6.30 (1H, s), 6.86 (1H, t, J = 10 Hz).

### Production Example 59: Preparation of Compound (173)

In the same manner as described in Production Example 55, except that 0.62 g of 3-chloro-1-propanol was used in place of methanol, 0.31 g of compound (173) was obtained.

¹H-NMR δ (ppm) [60 MHz, CDCl₃]: 2.13 (2H, t, J = 6 Hz), 3.30-3.46 (2H, m), 3.55 (3H, s), 3.67-3.85 (2H, m), 4.35 (2H, t, J = 6 Hz), 5.27 (1H, t, J = 8 Hz), 6.26 (1H, s), 6.84 (1H, t, J = 10 Hz).

### Production Example 60: Preparation of Compound (175)

In the same manner as described in Production Example 55, except that 0.98 g of 1-butanol was used in place of methanol, 0.09 g of compound (175) was obtained.

¹H-NMR δ (ppm) [60 MHz, CDCl₃]: 0.81-1.72 (7H, m), 3.27-3.43 (2H, m), 3.52 (3H, s), 4.17 (2H, t, J = 7 Hz), 5.24 (1H, t, J = 9 Hz), 6.24 (1H, s), 6.82 (1H, t, J = 10 Hz).

### Production Example 61: Preparation of Compound (181)

In 5 mi of xylene, 0.3 g of 1-(2-allyl-4-chloro-3-hydroxyphenyl)-3-methyl-4-trifluoromethyl-1,2,3,6-tetrahydropyrimidine-2,6-dione was dissolved, to which a catalytic amount of p-toluenesulfonic acid monohydrate was added, and the mixture was refluxed for 4 hours. After completion of the reaction, the reaction mixture was poured into water, and the mixture was extracted with ethyl acetate. The extract was washed with an aqueous solution of sodium hydrogencarbonate, dried and concentrated. The residue was purified by preparative thin layer chromatography, which afforded 0.3 g of compound (181).

¹H-NMR δ (ppm) [250 MHz, CDCl₃]: 1.52 (3H, d, J = 6.2 Hz), 2.73 (¹/₂H, dd, J = 4.1, 13 Hz), 2.77 (¹/₂H, dd, J = 3.9, 13 Hz), 3.20 (¹/₂H, dd, J = 3.1, 13 Hz), 3.23 (¹/₂H, dd, J = 3.1, 13 Hz), 3.55 (3H, s), 5.08 (1H, m), 6.35 (1H, s), 6.63 (1H, d, J = 8.9 Hz), 7.24 (1H, d, J = 8.9 Hz).

### Production Example 62: Preparation of Compound (194)

In 50 ml of chloroform, 4.0 g of 1-(2-allyl-4-chloro-3-hydroxyphenyl)-3-methyl-4-trifluoromethyl-1,2,3,6-tetrahydropyrimidine-2,6-dione was dissolved, to which 3.6 g of m-chloroperbenzoic acid was added at 5°C, and the reaction was effected at room temperature for 14 hours. After completion of the reaction, the reaction mixture was poured into an aqueous solution of sodium hydrogensulfite, and the mixture was extracted with ethyl acetate. The organic layer was washed with an aqueous solution of potassium carbonate and then water, dried and concentrated. The residue was purified by silica gel chromatography, which afforded 3.8 g of compound (194).

¹H-NMR δ (ppm) [250 MHz, CDCl₃]: 1.90 (1H, br), 2.92-3.12 (2H, m), 3.55 (1H, s), 3.76 (¹/₂H, dd, J = 5.4, 12 Hz), 3.77 (¹/₂H, dd, J = 5.7, 12 Hz), 3.88 (¹/₂H, dd, J = 7.7, 12 Hz), 3.89 (¹/₂H, dd, J = 7.5, 12 Hz), 5.0 (1H, m), 6.34 (¹/₂H, s), 6.35 (¹/₂H, s), 6.66 (1H, d, J = 8.5 Hz), 7.25 (1H, d, J = 8.5 Hz).

### Production Example 63: Preparation of Compound (195)

In 5 ml of N,N-dimethylformamide, 0.3 g of compound (194) was dissolved, to which 0.2 ml of methyl iodide and 0.1 g of sodium hydride (60% in oil) were added, and the mixture was stirred at room temperature for 20 hours. After completion of the reaction, the reaction mixture was poured into water, and the mixture was extracted with diethyl ether. The extract was dried and concentrated. The residue was purified by preparative thin layer chromatography, which afforded 0.19 g of compound (195).

¹H-NMR δ (ppm) [250 MHz, CDCl₃]: 3.0 (1H, m), 3.18 (1H, m), 3.41 (3H, s), 3.55 (3H, s), 3.57-3.71 (2H, m), 5.07 (1H, m), 6.35 (1H, s), 6.65 (1H, d, J = 8.5 Hz), 7.23 (1H, d, J = 8.5 Hz).

### Production Example 64: Preparation of Compound (198)

In 5 ml of chloroform, 0.3 g of compound (194) was dissolved, to which 0.5 ml of diisopropylethylamine and 0.15 g of chloromethyl methyl ether were added, and the mixture was stirred at room temperature for 18 hours. After completion of the reaction, the reaction mixture was poured into water, and the mixture was extracted with ethyl acetate. The organic layer was dried and concentrated. The residue was purified by preparative thin layer chromatography, which afforded 0.27 g of compound (198).

¹H-NMR δ (ppm) [300 MHz, CDCl₃]: 2.96 (¹/₂H, dd, J = 4.6, 16 Hz), 2.98 (¹/₂H, dd, J = 4.6, 16 Hz), 3.17 (¹/₂H, dd, J = 4.9, 15 Hz), 3.20 (¹/₂H, dd, J = 4.8, 15 Hz), 3.37 (3H, s), 3.55 (3H, s), 3.71-3.83 (2H, m), 4.67 (2H, s), 5.10 (1H, m), 6.35 (1H, s), 6.65 (1H, d, J = 8.5 Hz), 7.25 (1H, d, J = 8.5 Hz).

### Production Example 65: Preparation of Compound (201)

To 0.4 g of compound (194), 5 ml of pyridine and 3 ml of acetic anhydride were added, and the mixture was stirred at room temperature for 3 hours. After completion of the reaction, the reaction mixture was concentrated. The residue was purified by preparative thin layer chromatography, which afforded 0.37 g of compound (201).

¹H-NMR δ (ppm) [250 MHz, CDCl₃]: 2.08 (3H, s), 2.92 (1H, dd, J = 6.7, 15 Hz), 3.21 (¹/₂H, dd, J = 9.5, 15 Hz), 3.23 (¹/₂H, dd, J = 9.5, 15 Hz), 3.55 (3H, s), 4.33 (2H, d, J = 4.6 Hz), 5.12 (1H, m), 6.35 (1H, s), 6.67 (1H, d, J = 8.1 Hz), 7.27 (1H, d, J = 8.1 Hz).

### Production Example 66: Preparation of Compound (214)

In 20 ml of acetone, 1.8 g of compound (194) was dissolved, to which 3.0 ml of Jones reagent was added, and the mixture was stirred at room temperature for 4 hours. After completion of the reaction, the reaction mixture was filtered, and filtrate was poured into water. The mixture was extracted with ethyl acetate. The organic layer was dried and concentrated, which afforded 1.5 g of compound (214).

¹H-NMR δ (ppm) [250 MHz, CDCl₃]: 3.30-3.55 (2H, m), 3.58 (3H, s), 5.38 (1H, m), 6.40 (1H, s), 6.76 (1H, d, J = 8.5 Hz), 7.34 (1H, d, J = 8.5 Hz).

### Production Example 67: Preparation of Compound (215)

In 5 ml of methanol, 0.3 g of compound (214) was dissolved, to which a catalytic amount of p-toluenesulfonic acid monohydrate was added, and the mixture was heated under reflux for 4 hours. After completion of the reaction, the reaction mixture was poured into a saturated aqueous solution of sodium hydrogencarbonate, and the mixture was extracted with ethyl acetate. The organic layer was washed with water, dried and concentrated. The residue was applied to preparative thin layer chromatography, which afforded 0.21 g of compound (215).

¹H-NMR δ (ppm) [250 MHz, CDCl₃]: 3.30 (1H, m), 3.48 (1H, m), 3.55 (3H, s), 3,81 (3H, s), 5.34 (1H, dd, J = 7.2, 10.5 Hz), 6.34 (1H, s), 6.71 (1H, d, J = 8.6 Hz), 7.29 (1H, d, J = 8.6 Hz).

### Production Example 68: Preparation of Compound (218)

In the same manner as described in Production Example 67, except that 5 ml of 1-butanol was used in place of methanol, 0.2 g of compound (218) was obtained.

¹H-NMR δ (ppm) [250 MHz, CDCl₃]: 0.93 (3H, t, J = 7.5 Hz), 1.35 (2H, m), 1.65 (2H, m), 3.27 (1H, m), 3.45 (1H, m), 3.55 (3H, s), 4.21 (2H, t, J = 6.6 Hz), 5.31 (1H, dd, J = 8.3, 10.5 Hz), 6.35 (1H, s), 6.70 (1H, d, J = 8.5 Hz), 7.51 (1H, d, J = 8.5 Hz).

The following will describe several production examples of the starting material (II).

### Production Example 69: Preparation of 1-(2-allyl-4-chloro-6-fluoro-3-hydroxyphenyl)-3-methyl-4-trifluoromethyl-1,2,3,6-tetrahydropyrimidine-2,6-dione

In 30 ml of N,N-diethylaniline, 10.0 g of 1-(5-allyloxy-4-chloro-2-fluorophenyl)-3-methyl-4-trifluoromethyl-1,2,3,6-tetrahydropyrimidine-2,6-dione was dissolved, and the solution was refluxed for 3.5 hours. After completion of the reaction, the reaction mixture was poured into water, and the mixture was extracted with 300 ml of ethyl acetate. The extract was washed three times with 100 ml of 10% hydrochloric acid, dried and concentrated. The residue was purified by silica gel chromatography (eluent; hexane : ethyl acetate = 2 : 1), which afforded 7.9 g of the desired compound.

m.p., 148.0°C;

¹H-NMR δ (ppm) [60 MHz, CDCl₃]: 3.30 (2H, d, J = 6 Hz), 3.54 (3H, s), 4.75-4.90 (1H, m), 5.0-5.1 (1H, m), 5.5-5.9 (2H, m), 6.34 (1H, s), 7.12 (1H, d, J = 10 Hz).

### Production of Example 70: Preparation of 1-(2-allyl-4,6-difluoro-3-hydroxyphenyl)-3-methyl-4-trifluoromethyl-1,2,3,6-tetrahydropyrimidine-2,6-dione

In 10 ml of m-diisobutylbenzene, 2.0 g of 1-(5-allyloxy-2,4-difluorophenyl)-3-methyl-4-trifluoromethyl-1,2,3,6-tetrahydropyrimidine-2,6-dione was dissolved, and the solution was refluxed for 4 hours. After completion of the reaction, the reaction mixture was cooled to room temperature, and directly purified by silica gel chromatography (eluent; hexane : ethyl acetate = 2 : 1), which afforded 1.52 g of the desired compound.

m.p., 142.9°C;

¹H-NMR δ (ppm) [60 MHz, CDCl₃]: 3.21 (2H, d, J = 6 Hz), 3.49 (3H, s), 4.7-4.9 (1H, m), 4.9-5.1 (1H, m), 5.7-5.9 (2H, m), 6.25 (1H, s), 6.79 (1H, t, J = 10 Hz).

### Production Example 71: Preparation of 1-[4-chloro-6-fluoro-3-hydroxy-2-(9-methyl-2-propenyl)phenyl)-3-methyl-4-trifluoromethyl-1,2,3,6,-tetrahydropyrimidine-2,6-dione

In 50 ml of N,N-diethylaniline, 21.6 g of 1-[4-chloro-2-fluoro-5-(2-methyl-2-propenyloxy)phenyl]-3-methyl-4-trifluoromethyl-1,2,3,6-tetrahydropyrimidine-2,6,-dione was dissolved, and the solution was refluxed for 4 hours. After completion of the reaction, the reaction mixture was cooled to room temperature and extracted with diethyl ether. The extract was washed with diluted hydrochloric acid, dried and concentrated. The residue was purified by silica gel chromatography (eluent; hexane : ethyl acetate = 2 : 1), which afforded 19.5 g of the desired compound.

¹H-NMR δ (ppm) [60 MHz, CDCl₃]: 1.60 (3H, s), 3.28 (2H, s), 3.48 (3H, s), 4.4-4.6 (1H, m), 4.6-4.8 (1H, m), 5.76 (1H, s), 6.36 (1H, s), 7.09 (1H, d, J = 10 Hz).

### Production Example 72: Preparation of 1-(2-allyl-4-chloro-3-hydroxyphenyl)-3-methyl-4-trifluoromethyl-1,2,3,6-tetrahydropyrimidine-2,6,-dione

In 100 ml of N,N-diethylaniline, 8.0 g of 1-(3-allyloxy-4-chlorophenyl)-3-methyl-4-trifluoromethyl-1,2,3,6,-tetrahydropyrimidine-2,6,-dione was dissolved, and the solution was stirred at 180°C for 3 hours. After completion of the reaction, the reaction mixture was cooled to room temperature and poured into diluted hydrochloric acid. The mixture was extracted with ethyl acetate. The organic layer was washed with water, dried and concentrated. The residue was purified by silica gel chromatography, which afforded 6.3 g of the desired compound.

¹H-NMR δ (ppm) [60 MHz, CDCl₃]: 3.15 (1H, s), 3.25 (1H, s), 3.42 (3H, s), 4.65-5.05 (2H, m), 5.65 (1H, m), 5.80 (1H, s), 6.25 (1H, s), 6.57 (1H, d, J = 8 Hz), 7.25 (1H, d, J = 8 Hz).

### Production Example 73: Preparation of 1-[4-chloro-3-hydroxy-2-(2-methyl-2-propenyl)phenyl]-3-methyl-4-trifluoromethyl-1,2,3,6,-tetrahydropyrimidine-2,6-dione

In 150 ml of N,N-dimethylaniline, 7.4 g of 1-[4-chloro-3-(2-methyl-2-propenyloxy)phenyl]-3-methyl-4-trifluoromethyl-1,2,3,6,-tetrahydropyrimidine-2,6-dione was dissolved, and the solution was stirred at 160°C for 3 hours. After completion of the reaction, the reaction mixture was cooled to room temperature and poured into water. The mixture was extracted with diethyl ether. The organic layer was washed with dilute hydrochloric acid, dried and concentrated, which afforded 6.2 g of the desired compound.

¹H-NMR δ (ppm) [60 MHz, CDCl₃]: 1.58 (3H, s), 3.28 (2H, s), 3.46 (3H, s), 4.45 (1H, s), 4.58 (1H, s), 6.14 (1H, s), 6.26 (1H, s), 6.60 (1H, d, J = 8 Hz), 7.26 (1H, d, J = 8 Hz).

The following will describe a production example of compound (IV).

### Production Example 74

In 500 ml of chloroform, 50 g of 1-[4-chloro-6-fluoro-3-hydroxy-2-(2-methyl-2-propenyl)phenyl]-3-methyl-4-trifluoromethyl-1,2,3,6-tetrahydropyrimidine-2,6-dione was dissolved, to which 30.0 g of m-chloroperbenzoic acid was added, and the reaction was effected at 60°C for 4.5 hours. After completion of the reaction, the reaction mixture was cooled to room temperature and subjected to fractionation with a separatory funnel using chloroform and an aqueous solution of sodium sulfite. The organic layer was washed with a saturated aqueous solution of sodium hydrogencarbonate and then water, dried and concentrated. The residue was subjected to recrystallization, which afforded 46 g of 1-[4-chloro-6-fluoro-3-hydroxy-2-(2,3-epoxy-2-methylpropyl)phenyl]-3-methyl-4-trifluoromethyl-1,2,3,6-tetrahydropyrimidine-2,6-dione.

¹H-NMR δ (ppm) [60 MHz, CDCl₃]: 1.29 (½ x 3H, s), 1.31 (½ x 3H, s), 2.75 (3H, m), 3.10 (1H, m), 3.58 (3H, s), 6.31 (1H, s), 7.20 (1H, d, J = 8 Hz), 7.45 (1H, s).

The following will describe formulation examples wherein parts are by weight. The compounds of the present invention are designated by the corresponding numbers as shown in Table 1.

### Formulation Example 1

Fifty parts of any one of the compounds (1), (4), (11), (13)-(17), (23)-(25), (28), (29), (32), (33), (36), (38), (39), (44), (49), (50), (53), (56), (70), (72), (73), (75), (101), (104), (105), (107), (108), (111)-(117), (120)-(129), (131)-(134), (138), (139), (142), (143), (147), (150), (152)-(155), (157)-(158), (162), (163), (165)-(169), (171)-(175), (178), (180), (194), (195), (198), (201), 3 parts of calcium ligninsulfonate, 2 parts of sodium laurylsulfate and 45 parts of synthetic hydrous silicate are well mixed while being powdered to obtain a wettable powder.

### Formulation Example 2

Ten parts of any one of the compounds (1), (4), (11), (13)-(17), (23)-(25), (28), (29), (32), (33), (36), (38), (39), (44), (49), (50), (53), (56), (70), (72), (73), (75), (101), (104), (105), (107), (108), (111)-(117), (120)-(129), (131)-(134), (138), (139), (142), (143), (147), (150), (152)-(155), (157)-(158), (162), (163), (165)-(169), (171)-(175), (178), (180), (194), (195), (198), (201), 14 parts of polyoxyethylene styryl phenyl ether, 6 parts of calcium dodecylbenzenesulfonate, 35 parts of xylene and 35 parts of cyclohexanone are well mixed to obtain an emulsifiable concentrate.

### Formulation Example 3

Two parts of any one of the compounds (1), (4), (11), (13)-(17), (23)-(25), (28), (29), (32), (33), (36), (38), (39), (44), (49), (50), (53), (56), (70), (72), (73), (75), (101), (104), (105), (107), (108), (111)-(117), (120)-(129), (131)-(134), (138), (139), (142), (143), (147), (150), (152)-(155), (157)-(158), (162), (163), (165)-(169), (171)-(175), (178), (180), (194), (195), (198), (201), 2 parts of synthetic hydrous silicate, 2 parts of calcium ligninsulfonate, 30 parts of bentonite and 64 parts of kaoline clay are well mixed while being powdered. The mixture is then kneaded with water, granulated and dried to obtain granules.

### Formulation Example 4

Twenty five parts of any one of the compounds (1), (4), (11), (13), (14), (16), (17), (23), (24), (25), (28), (29), (32), (33), (38), (39), (49), (70), (101), (104), (105), (107), (108), (111), (112), (113), (114), (120), (121), (123), (124), (126), (127), (132), (133), (139), (150), (152), (153), (158), (162), (163), (166), (167), (168), (172), (173), (194), (201), 50 parts of 10% solution of polyvinylalcohol and 25 parts of water are well mixed, and the mixture was then pulverized until the particle size thereof becomes not greater than 5 microns to obtain a flowable.

The following will describe test examples. The compounds of the present invention are designated by the corresponding numbers as shown in Table 1, and the compounds used for comparison are designated by the numbers as shown in Table 2.

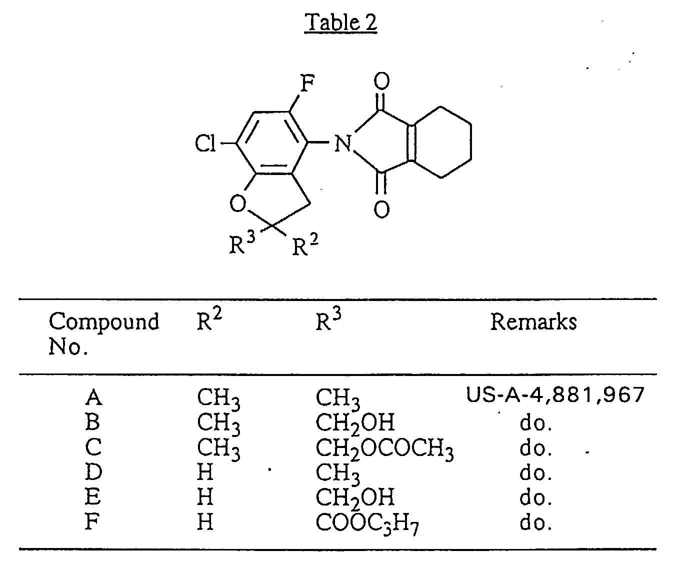

The herbicidal activity on weeds and the phytotoxicity to crop plants were determined by visual observation as to the degree of germination and growth of the test plants (i.e., weeds and crop plants), and rated with an index 0, 1, 2, 3, 4 or 5, the numeral "0" indicating little or no difference as seen in comparison with the untreated plants and the numeral "5" indicating the complete death of the test plants or the complete inhibition of their germination or growth.

### Test Example 1

Cylindrical plastic pots (diameter, 10 cm; height, 10 cm) were filled with upland field soil, and the seeds of wild oat and tall morningglory were sowed therein and covered with soil. The test compound formulated in the emulsifiable concentrate according to the Formulation Example 2 was diluted with water, and the dilution was sprayed onto the soil surface by an automatic sprayer at an application amount of 1000 liters per hectare. The test plants were grown in a greenhouse for 19 days, and the herbicidal activity was examined. The results are shown in Table 3.

**Table 3**

| Compound No. | Dosage (a.i. g/ha) | Herbicidal activity | |
|---|---|---|---|
| | | Wild oat | Tall morningglory |
| (1) | 31 | 5 | 5 |
| (4) | 31 | 5 | 5 |
| (11) | 31 | 5 | 5 |
| (13) | 31 | 5 | 5 |
| (14) | 31 | 5 | 5 |
| (15) | 31 | 5 | 5 |
| (16) | 31 | 5 | 4 |
| (17) | 31 | 5 | 5 |
| (29) | 31 | 4 | 4 |
| (101) | 31 | 5 | 5 |
| (104) | 31 | 5 | 5 |
| (105) | 31 | 5 | 4 |
| (111) | 31 | 5 | 5 |
| (112) | 31 | 5 | 5 |
| (114) | 31 | 4 | 5 |
| (115) | 31 | 5 | 5 |
| (116) | 31 | 5 | 5 |
| (117) | 31 | 4 | 5 |
| (121) | 31 | 4 | 4 |
| (122) | 31 | 4 | 5 |
| (139) | 31 | 5 | 5 |
| (147) | 31 | 4 | 4 |
| A | 31 | 1 | 1 |
| B | 31 | 3 | 2 |
| C | 31 | 3 | 1 |
| D | 31 | 0 | 0 |
| E | 31 | 0 | 1 |
| F | 31 | 0 | 0 |

### Test Example 2

Cylindrical plastic pots (diameter, 10 cm; height, 10 cm) were filled with upland field soil, and seeds of wild oat were sowed therein and cultivated in a greenhouse for 7 days. After that, the test compound formulated in the emulsifiable concentrate according to the Formulation Example 2 was diluted with water containing a spreading agent, and the dilution was sprayed over the foliage of the test plants by an automatic sprayer at an application amount of 1000 liters per hectare. After the treatment, the test plants were further grown in the greenhouse for 19 days, and the herbicidal activity was examined. The results are shown in Table 4.

**Table 4**

| Compound No. | Dosage (a.i. g/ha) | Herbicidal activity |
|---|---|---|
| | | Wild oat |
| (1) | 31 | 5 |
| (11) | 31 | 4 |
| (13) | 31 | 4 |
| (15) | 31 | 4 |
| (16) | 31 | 5 |
| (23) | 31 | 5 |
| (24) | 31 | 5 |
| (25) | 31 | 4 |
| (28) | 31 | 5 |
| (29) | 31 | 5 |
| (32) | 31 | 5 |
| (33) | 31 | 5 |
| (36) | 31 | 5 |
| (38) | 31 | 5 |
| (101) | 31 | 5 |
| (105) | 31 | 4 |
| (107) | 31 | 4 |
| (108) | 31 | 4 |
| (115) | 31 | 5 |
| (125) | 31 | 5 |
| (128) | 31 | 4 |
| (129) | 31 | 5 |
| (134) | 31 | 4 |
| A | 31 | 2 |
| C | 31 | 2 |
| D | 31 | 1 |
| F | 31 | 2 |

### Test Example 3

Plastic pots (25 cm x 18 cm x 7 cm) were filled with upland field soil, and the seeds of soybean, tall momingglory, bamyardgrass and Johnsongrass were sowed therein and covered with soil. The test compound formulated in an emulsifiable concentrate according to the Formulation Example 2 was diluted with water, and the dilution was sprayed onto the soil surface by an automatic sprayer at an application amount of 1000 liters per hectare. The test plants were grown in a greenhouse for 18 days, and the herbicidal activity and the phytotoxicity were examined. The results are shown in Table 5.

**Table 5**

| Compound No. | Dosage (a.i. g/ha) | Phytotoxicity | Herbicidal activity | | |
|---|---|---|---|---|---|
| | | Soybean | Tall morningglory | Barnyardgrass | Johnsongrass |
| (23) | 31 | 0 | 5 | 4 | 4 |
| (28) | 125 | 1 | 5 | 5 | 5 |
| | 31 | 0 | 5 | 5 | 5 |
| (29) | 125 | 1 | 4 | 5 | 5 |
| (32) | 31 | 0 | 4 | 5 | 4 |
| (53) | 63 | 1 | 4 | 5 | 5 |
| (70) | 125 | 1 | 5 | 5 | 4 |
| (123) | 125 | 1 | 4 | 4 | 4 |
| (128) | 125 | 0 | 5 | 5 | 5 |
| (129) | 125 | 0 | 5 | 5 | 5 |
| (134) | 125 | 1 | 5 | 5 | 5 |
| A | 125 | 1 | 3 | 3 | 4 |
| | 31 | 0 | 1 | 1 | 2 |
| D | 125 | 0 | 0 | 3 | 0 |
| F | 125 | 0 | 3 | 1 | 1 |
| | 31 | 0 | 1 | 0 | 0 |

### Test Example 4

Plastic pots (25cm x 18 cm x 7 cm) were filled with upland field soil, and the seeds of wheat, catchweed bedstraw, birdseye speedwell and field pansy were sowed therein, and cultivated in a greenhouse for 29 days. The test compound formulated in the emulsifiable concentrate according to the Formulation Example 2 was diluted with water, and the dilution was sprayed over the foliage of the test plants by an automatic sprayer at an application amount of 1000 liters per hectare. The test plants were further grown in the greenhouse for 25 days, and the herbicidal activity and crop phytotoxicity were examined. At the time of the application, the test plants were generally at 1 to 4 leaf stage and in 3-25 cm height, although the growing stage of the test plants varied depending on their species. The results are shown in Table 6.

**Table 6**

| Compound No. | Dosage (a.i. g/ha) | Phytotoxicity | Herbicidal activity | | |
|---|---|---|---|---|---|
| | | Wheat | Catchweed bedstraw | Birdseye speedwell | Field pansy |
| (1) | 16 | 1 | 5 | 5 | 5 |
| (4) | 16 | 1 | 5 | 5 | 5 |
| (11) | 16 | 0 | 5 | 5 | 5 |
| (14) | 16 | 1 | 4 | 4 | 5 |
| (15) | 16 | 1 | 5 | 5 | 5 |
| (16) | 16 | 1 | 4 | | 5 |
| (17) | 16 | 1 | 4 | 5 | 5 |
| (23) | 16 | 1 | 4 | 5 | 5 |
| (24) | 4 | 1 | 5 | 5 | 5 |
| (25) | 16 | 0 | 5 | 5 | 5 |
| | 4 | 0 | 4 | 5 | 4 |
| (28) | 16 | 1 | 5 | 5 | 5 |
| (29) | 16 | 1 | 5 | 5 | 5 |
| (32) | 16 | 1 | 5 | 5 | 5 |
| | 4 | 1 | 5 | 4 | 5 |
| (33) | 16 | 1 | 5 | 5 | 5 |
| (36) | 16 | 1 | 4 | 5 | 5 |
| (38) | 16 | 1 | 4 | 5 | 5 |
| | 4 | 1 | 4 | 5 | 5 |
| (70) | 16 | 0 | 5 | 5 | 5 |
| (101) | 4 | 0 | - | 4 | 4 |
| (104) | 31 | 0 | 5 | 5 | 5 |
| | 8 | 0 | 5 | 5 | 4 |
| (108) | 16 | 0 | 4 | 5 | 5 |
| (111) | 31 | 1 | 5 | 5 | 5 |
| | 8 | 0 | 5 | 5 | 4 |
| (112) | 31 | 1 | 5 | 4 | 5 |
| (115) | 31 | 1 | 5 | 5 | 5 |
| (116) | 31 | 1 | 4 | 5 | 5 |
| | 8 | 0 | 4 | 4 | 5 |
| (117) | 31 | 1 | 5 | 4 | 5 |
| (131) | 31 | 1 | 5 | 5 | 4 |
| (132) | 31 | 1 | 5 | 5 | 5 |
| (133) | 31 | 1 | 5 | 5 | 4 |
| (139) | 31 | 1 | 5 | 4 | 4 |
| (155) | 16 | 1 | 4 | 4 | 4 |
| (175) | 16 | 1 | 4 | 5 | 4 |
| A | 31 | 1 | 3 | 4 | 4 |
| | 8 | 1 | 3 | 3 | 3 |
| B | 31 | 2 | 3 | 3 | 5 |
| | 8 | 1 | 3 | 1 | 3 |
| C | 31 | 2 | 4 | 3 | 4 |
| | 8 | 1 | 3 | 1 | 3 |
| D | 31 | 1 | 2 | 1 | 2 |
| E | 31 | 1 | 2 | 5 | 5 |
| | 8 | 1 | 2 | 3 | 4 |
| F | 31 | 1 | 3 | 3 | 2 |
| | 8 | 1 | 3 | 2 | 1 |

### Test Example 5

Plastic pots (19 cm x 29 cm x 8 cm) were filled with upland field soil, and the seeds of cotton were sowed therein, and cultivated in a greenhouse for 3 months. The test compound formulated in the emulsifiable concentrate according to the Formulation Example 2 was diluted with water containing a spreading agent, and the dilution was sprayed over the foliage of the test plant by a spray machine at an application amount of 1000 liters per hectare. After the treatment, the test plants were further grown in the greenhouse for 9 days, and the desiccating effect of the foliage of the cotton plants was examined. At the time of the application, the test plants were in 50 cm height and began to set bolls. The desiccating effect on cotton foliage was determined by 0-100 (%), the numeral "0" indicating no difference in comparison with untreated plant and the numeral "100" indicating the complete desiccating effect. The results are shown in Table 7.

**Table 7**

| Compound No. | Dosage (a.i. g/ha) | Desiccating effect (%) |
|---|---|---|
| (24) | 100 | 93 |

### Test Example 6

Plastic pots (20 cm x 30 cm x 8 cm depth) were filled with upland field soil, and seeds of ivyleaf momingglory, cleavers and giant foxtail were sowed therein. Each of test compounds formulated into emulsifiable concentrate according to Formulation Example 2 was diluted with water, and each of the dilution was sprayed onto the soil surface of the test pot by a spray machine. The application dosage of each of the test compound was 250 grams per hectare and application amount was 1000 liters per hectare. After the treatment, the test plants were grown in a greenhouse for 25 days and each weed control was examined. The results are shown in Table 8.

**Table 8**

| Compound No. | Dosage (a.i. g/ha) | Herbicidal activity | | |
|---|---|---|---|---|
| | | Ivyleaf morningglory | Cleavers | Giant foxtail |
| (181) | 250 | 5 | 5 | 5 |
| (194) | 250 | 5 | 5 | 5 |
| (195) | 250 | 5 | 5 | 5 |
| (198) | 250 | 5 | 5 | 5 |
| (201) | 250 | 5 | 5 | 5 |
| (216) | 250 | 5 | 5 | 5 |

### Test Example 7

Plastic pots (20 cm x 30 cm x 8 cm depth) were filled with upland field soil, and seeds of ivyleaf morningglory, pale smartweed, velvetleaf, barnyardgrass and wild oat were sowed therein, and cultivated in a greenhouse for 31 days. Each of test compounds formulated into emulsifiable concentrate according to Formulation Example 2 was diluted with water containing a spreading agent, and each of the dilution was sprayed over the foliage of the test plants by a spray machine. The application dosage of each of the test compound was 250 grams per hectare and application amount was 1000 liters per hectare. At the time of the application, the test plants were in approximately 5 to 20 cm height. After the treatment, the test plants were grown in a greenhouse for 25 days and each weed control was examined. The results are shown in Table 9.

**Table 9**

| Compound No. | Dosage (a.i. g/ha) | Herbicidal activity | | | | |
|---|---|---|---|---|---|---|
| | | Ivyleaf morningglory | Pale smart weed | Velvetleaf | Barnyard grass | Wild oat |
| (181) | 250 | 5 | 5 | 5 | 5 | 5 |
| (195) | 250 | 5 | 5 | 5 | 5 | 5 |
| (198) | 250 | 5 | 5 | 5 | 5 | 5 |
| (214) | 250 | 5 | 5 | 5 | 5 | 5 |
| (215) | 250 | 5 | 5 | 5 | 5 | 5 |
| (216) | 250 | 5 | 5 | 5 | 5 | 5 |
| (218) | 250 | 5 | 5 | 5 | 5 | 5 |
| (242) | 250 | 5 | 5 | 5 | 5 | 5 |

As described above, the compounds (I) of the present invention have excellent herbicidal activity against various unfavorable weeds under the soil treatment or foliar treatment on upland fields and under the flooding treatment on paddy fields, and some of them exhibit excellent selectivity between crops and weeds; therefore, they are useful as active ingredients of herbicides.

## Claims (Claims for the following Contracting State(s): DE, ES, FR, GB, IT)

1. A compound of the formula: 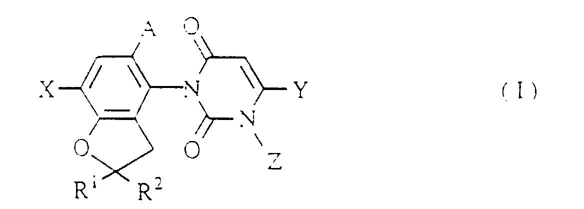 wherein A is hydrogen, fluorine or chlorine; X is hydrogen, fluorine, chlorine or bromine; Y is methyl optionally substituted with one or more halogen atoms; Z is methyl or amino; R¹ is hydrogen or C₁-C₆ alkyl; and R² is C₁-C₆ alkyl, C₁-C₆ haloalkyl, C₁-C₆ hydroxyalkyl, C₁-C₆ alkoxy(C₁-C₆)alkyl, C₁-C₆ alkoxy(C₁-C₆)alkoxy(C₁-C₆)-alkyl, C₁-C₇ acyloxy(C₁-C₆)alkyl, carboxyl, C₁-C₆ alkoxycarbonyl, C₁-C₆ haloalkoxycarbonyl, C₃-C₆ cycloalkoxycarbonyl, C₂-C₆ alkynyloxycarbonyl, aminocarbonyl, C₁-C₆ alkylaminocarbonyl or phenylaminocarbonyl having a phenyl group optionally substituted with C₁-C₆ alkyl, C₁-C₆ alkoxy, C₁-C₆ haloalkyl, C₁-C₆ haloalkoxy, halogen, nitro, cyano or C₁-C₆ alkylthio; provided that if R¹ is hydrogen and A is fluorine or chlorine, X must be different from chlorine and bromine.

2. A compound according to claim 1, wherein R¹ is C₁-C₃ alkyl.

3. A compound according to claim 1, wherein R¹ is methyl.

4. A compound according to claim 1, 2 or 3 wherein A is hydrogen.

5. A herbicidal composition comprising, as an active ingredient, a compound according to any of claims 1 to 4, and an inert carrier or diluent.

6. A method for exterminating unfavorable weeds, which comprises applying a compound according to any of claims 1 to 4 to an area where the unfavorable weeds grow or will grow.

7. Use of the compound according to claim 1 as a herbicide.

8. A compound of the formula: 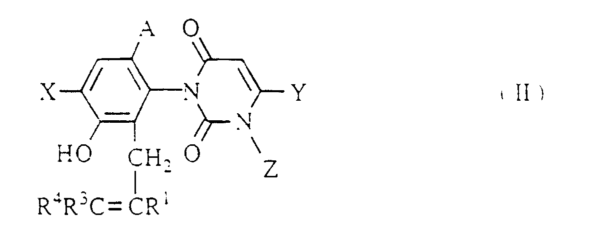 wherein A is hydrogen, fluorine or chlorine; X is hydrogen, fluorine, chlorine or bromine; Y is methyl optionally substituted with one or more halogen atoms; Z is methyl or amino; R¹ is hydrogen or C₁-C₆ alkyl; and R³ and R⁴ are the same or different and are hydrogen or C₁-C₆ alkyl with the proviso that the total number of carbon atoms in the R³ and R⁴ is not greater than 6.

9. A compound of the formula: 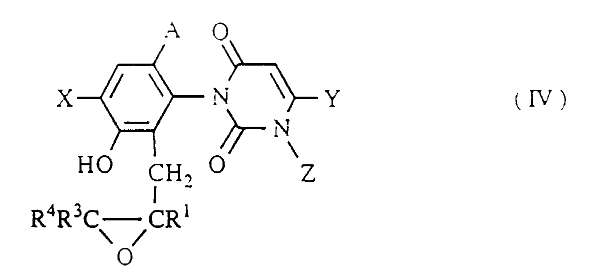 wherein A is hydrogen, fluorine or chlorine; X is hydrogen, fluorine, chlorine or bromine; Y is methyl optionally substituted with one or more halogen atoms; Z is methyl or amino; R¹ is hydrogen or C₁-C₆ alkyl; and R³ and R⁴ are the same or different and are hydrogen or C₁-C₆ alkyl with the proviso that the total number of carbon atoms in the R³ and R⁴ is not greater than 6.

## Claims (Claims for the following Contracting State(s): AT, CH, LI, GR)

1. A compound of the formula: 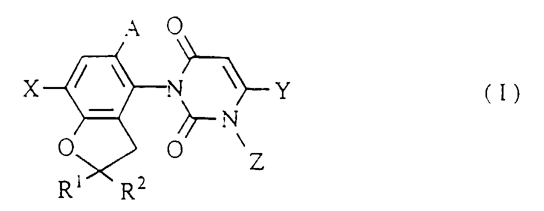 wherein A is hydrogen, fluorine or chlorine; X is hydrogen, fluorine, chlorine or bromine; Y is methyl optionally substituted with one or more halogen atoms; Z is methyl or amino; R¹ is hydrogen or C₁-C₆ alkyl; and R² is C₁-C₆ alkyl, C₁-C₆ haloalkyl, C₁-C₆ hydroxyalkyl, C₁-C₆ alkoxy(C₁-C₆)alkyl, C₁-C₆ alkoxy(C₁-C₆)alkoxy(C₁-C₆)-alkyl, C₁-C₇ acyloxy(C₁-C₆)alkyl, carboxyl, C₁-C₆ alkoxycarbonyl, C₁-C₆ haloalkoxycarbonyl, C₃-C₆ cycloalkoxycarbonyl, C₂-C₆ alkynyloxycarbonyl, aminocarbonyl, C₁-C₆ alkylaminocarbonyl or phenylaminocarbonyl having a phenyl group optionally substituted with C₁-C₆ alkyl, C₁-C₆ alkoxy, C₁-C₆ haloalkyl, C₁-C₆ haloalkoxy, halogen, nitro, cyano or C₁-C₆ alkylthio.

2. A compound according to claim 1, wherein R¹ is C₁-C₃ alkyl.

3. A compound according to claim 1, wherein R¹ is methyl.

4. A compound according to claim 1, 2 or 3 wherein A is hydrogen.

5. A herbicidal composition comprising, as an active ingredient, a compound according to any of claims 1 to 4, and an inert carrier or diluent.

6. A method for exterminating unfavorable weeds, which comprises applying a compound according to any of claims 1 to 4 to an area where the unfavorable weeds grow or will grow.

7. Use of the compound according to claim 1 as a herbicide.

8. A compound of the formula: 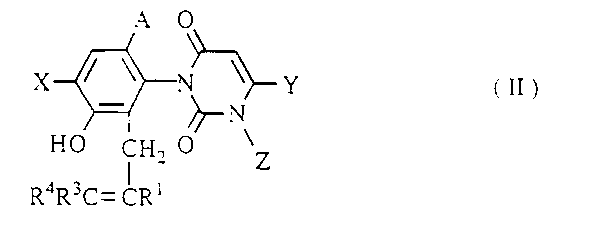 wherein A is hydrogen, fluorine or chlorine; X is hydrogen, fluorine, chlorine or bromine; Y is methyl optionally substituted with one or more halogen atoms; Z is methyl or amino; R¹ is hydrogen or C₁-C₆ alkyl; and R³ and R⁴ are the same or different and are hydrogen or C₁-C₆ alkyl with the proviso that the total number of carbon atoms in the R³ and R⁴ is not greater than 6.

9. A compound of the formula: 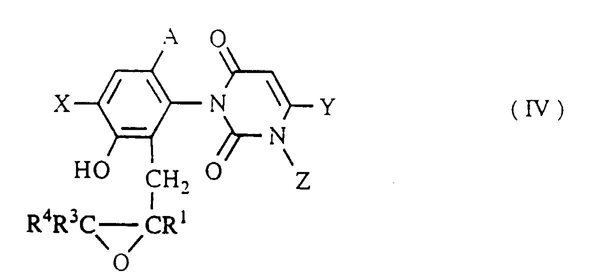 wherein A is hydrogen, fluorine or chlorine; X is hydrogen, fluorine, chlorine or bromine; Y is methyl optionally substituted with one or more halogen atoms; Z is methyl or amino; R¹ is hydrogen or C₁-C₆ alkyl; and R³ and R⁴ are the same or different and are hydrogen or C₁-C₆ alkyl with the proviso that the total number of carbon atoms in the R³ and R⁴ is not greater than 6.

## Patentansprüche (Patentansprüche für folgende(n) Vertragsstaat(en): DE, ES, FR, GB, IT)

1. Verbindung der Formel: 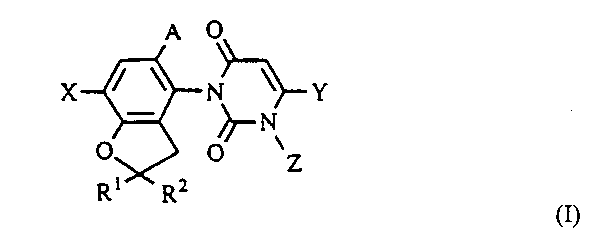 in der
- A ein Wasserstoff-, Fluor- oder Chloratom bedeutet;
- X ein Wasserstoff-, Fluor-, Chlor- oder Bromatom bedeutet;
- Y eine Methylgruppe bedeutet, die gegebenenfalls mit einem oder mehreren Halogenatomen substituiert ist;
- Z eine Methyl- oder Aminogruppe bedeutet;
- R¹ ein Wasserstoffatom oder einen (C₁-C₆)-Alkylrest bedeutet; und
- R² einen (C₁-C₆)-Alkyl-, (C₁-C₆)-Halogenalkyl-, (C₁-C₆)-Hydroxyalkyl-, (C₁-C₆)-Alkoxy-(C₁-C₆)-alkyl-, (C₁-C₆)-Alkoxy-(C₁-C₆)-alkoxy-(C₁-C₆)-alkyl-, (C₁-C₇)-Acyloxy-(C₁-C₆)-alkyl-, Carboxyl-, (C₁-C₆)-Alkoxycarbonyl-, (C₁-C₆)-Halogenalkoxycarbonyl-, (C₃-C₆)-Cycloalkoxycarbonyl-, (C₂-C₆)-Alkinyloxycarbonyl-, Aminocarbonyl-, (C₁-C₆)-Alkylaminocarbonyl- oder Phenylaminocarbonylrest bedeutet, wobei eine Phenylgruppe gegebenenfalls mit einem (C₁-C₆)-Alkyl-, (C₁-C₆)-Alkoxy-, (C₁-C₆)-Halogenalkyl-, (C₁-C₆)-Halogenalkoxy-, Halogen-, Nitro-, Cyano- oder (C₁-C₆)-Alkylthiorest substituiert ist,
mit der Maßgabe, daß, wenn R¹ ein Wasserstoffatom ist, und A ein Fluor- oder Chloratom ist, X von einem Chlor- und Bromatom verschieden sein muß.

2. Verbindung nach Anspruch 1, in der R¹ ein (C₁-C₃)-Alkylrest ist.

3. Verbindung nach Anspruch 1, in der R¹ eine Methylgruppe ist.

4. Verbindung nach Anspruch 1, 2 oder 3, in der A ein Wasserstoffatom ist.

5. Herbizidzusammensetzung, die als einen Wirkstoff eine Verbindung nach einem der Ansprüche 1 bis 4 sowie einen inerten Träger oder ein Verdünnungsmittel umfaßt.

6. Verfahren zur Vertilgung unvorteilhafter Unkräuter, umfassend Aufbringen einer Verbindung nach einem der Ansprüche 1 bis 4 auf eine Fläche, auf der die unvorteilhaften Unkräuter wachsen oder wachsen werden.

7. Verwendung der Verbindung nach Anspruch 1 als ein Herbizid.

8. Verbindung der Formel: 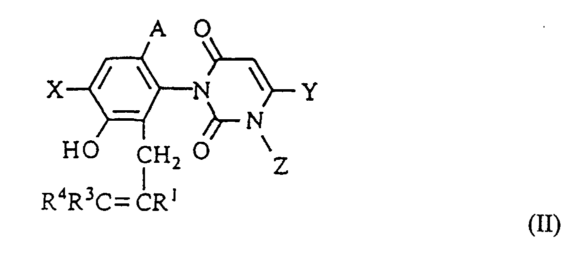 in der
- A ein Wasserstoff-, Fluor- oder Chloratom bedeutet;
- X ein Wasserstoff-, Fluor-, Chlor- oder Bromatom bedeutet;
- Y eine Methylgruppe bedeutet, die gegebenenfalls mit einem oder mehreren Halogenatomen substituiert ist;
- Z eine Methyl- oder Aminogruppe bedeutet;
- R¹ ein Wasserstoffatom oder einen (C₁-C₆)-Alkylrest bedeutet; und
- R³ und R⁴ gleich oder verschieden sind und Wasserstoffatome oder (C₁-C₆)-Alkylreste bedeuten, mit der Maßgabe, daß die Gesamtzahl der Kohlenstoffatome in den Resten R³ und R⁴ nicht größer als 6 ist.

9. Verbindung der Formel: 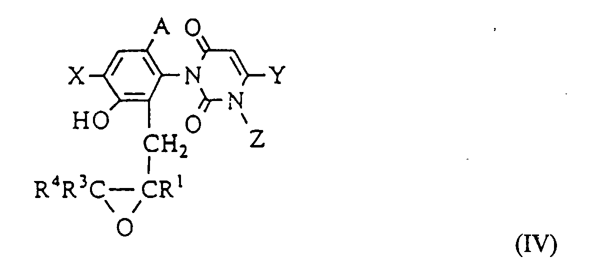 in der
- A ein Wasserstoff-, Fluor- oder Chloratom bedeutet;
- X ein Wasserstoff-, Fluor-, Chlor- oder Bromatom bedeutet;
- Y eine Methylgruppe bedeutet, die gegebenenfalls mit einem oder mehreren Halogenatomen substituiert ist;
- Z eine Methyl- oder Aminogruppe bedeutet;
- R¹ ein Wasserstoffatom oder einen (C₁-C₆)-Alkylrest bedeutet; und
- R³ und R⁴ gleich oder verschieden sind und Wasserstoffatome oder (C₁-C₆)-Alkylreste bedeuten, mit der Maßgabe, daß die Gesamtzahl der Kohlenstoffatome in den Resten R³ und R⁴ nicht größer als 6 ist.

## Patentansprüche (Patentansprüche für folgende(n) Vertragsstaat(en): AT, CH, LI, GR)

1. Verbindung der Formel: 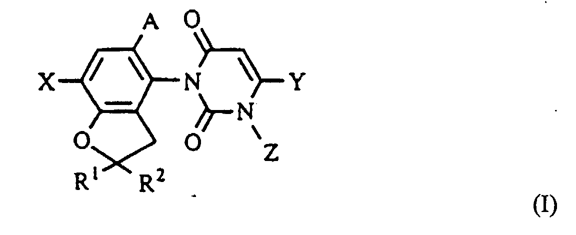 in der
- A ein Wasserstoff-, Fluor- oder Chloratom bedeutet;
- X ein Wasserstoff-, Fluor-, Chlor- oder Bromatom bedeutet;
- Y eine Methylgruppe bedeutet, die gegebenenfalls mit einem oder mehreren Halogenatomen substituiert ist;
- Z eine Methyl- oder Arninogruppe bedeutet;
- R¹ ein Wasserstoffatom oder einen (C₁-C₆)-Alkylrest bedeutet; und
- R² einen (C₁-C₆)-Alkyl-, (C₁-C₆)-Halogenalkyl-, (C₁-C₆)-Hydroxyalkyl-, (C₁-C₆)-Alkoxy-(C₁-C₆)-alkyl-, (C₁-C₆)-Alkoxy-(C₁-C₆)-alkoxy-(C₁-C₆)-alkyl-, (C₁-C₇)-Acyloxy-(C₁-C₆)-alkyl-, Carboxyl-, (C₁-C₆)-Alkoxycarbonyl-, (C₁-C₆)-Halogenalkoxycarbonyl-, (C₃-C₆)-Cycloalkoxycarbonyl-, (C₂-C₆)-Alkinyloxycarbonyl-, Aminocarbonyl-, (C₁-C₆)-Alkylaminocarbonyl- oder Phenylaminocarbonylrest bedeutet, wobei eine Phenylgruppe gegebenenfalls mit einem (C₁-C₆)-Alkyl-, (C₁-C₆)-Alkoxy-, (C₁-C₆)-Halogenalkyl-, (C₁-C₆)-Halogenalkoxy-, Halogen-, Nitro-, Cyano- oder (C₁-C₆)-Alkylthiorest substituiert ist.

2. Verbindung nach Anspruch 1, in der R¹ ein (C₁-C₃)-Alkylrest ist.

3. Verbindung nach Anspruch 1, in der R¹ eine Methylgruppe ist.

4. Verbindung nach Anspruch 1, 2 oder 3, in der A ein Wasserstoffatom ist.

5. Herbizidzusammensetzung, die als einen Wirkstoff eine Verbindung nach einem der Ansprüche 1 bis 4 sowie einen inerten Träger oder ein Verdünnungsmittel umfaßt.

6. Verfahren zur Vertilgung unvorteilhafter Unkräuter, umfassend Aufbringen einer Verbindung nach einem der Ansprüche 1 bis 4 auf eine Fläche, auf der die unvorteilhaften Unkräuter wachsen oder wachsen werden.

7. Verwendung der Verbindung nach Anspruch 1 als ein Herbizid.

8. Verbindung der Formel: 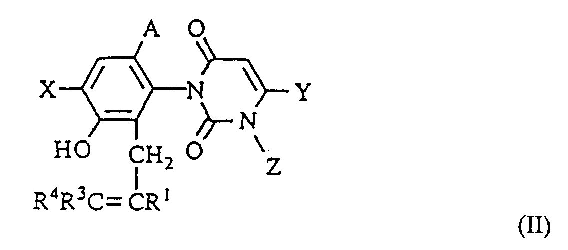 in der
- A ein Wasserstoff-, Fluor- oder Chloratom bedeutet;
- X ein Wasserstoff-, Fluor-, Chlor- oder Bromatom bedeutet;
- Y eine Methylgruppe bedeutet, die gegebenenfalls mit einem oder mehreren Halogenatomen substituiert ist;
- Z eine Methyl- oder Aminogruppe bedeutet;
- R¹ ein Wasserstoffatom oder einen (C₁-C₆)-Alkylrest bedeutet; und
- R³ und R⁴ gleich oder verschieden sind und Wasserstoffatome oder (C₁-C₆)-Alkylreste bedeuten, mit der Maßgabe, daß die Gesamtzahl der Kohlenstoffatome in den Resten R³ und R⁴ nicht größer als 6 ist.

9. Verbindung der Formel: 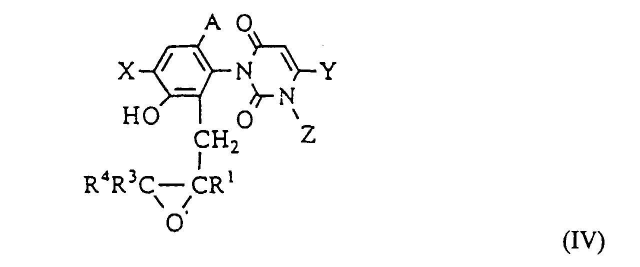 in der
- A ein Wasserstoff-, Fluor- oder Chloratom bedeutet;
- X ein Wasserstoff-, Fluor-, Chlor- oder Bromatom bedeutet;
- Y eine Methylgruppe bedeutet, die gegebenenfalls mit einem oder mehreren Halogenatomen substituiert ist;
- Z eine Methyl- oder Aminogruppe bedeutet;
- R¹ ein Wasserstoffatom oder einen (C₁-C₆)-Alkylrest bedeutet; und
- R³ und R⁴ gleich oder verschieden sind und Wasserstoffatome oder (C₁-C₆)-Alkylreste bedeuten, mit der Maßgabe, daß die Gesamtzahl der Kohlenstoffatome in den Resten R³ und R⁴ nicht größer als 6 ist.

## Revendications (Revendications pour l'(les) Etat(s) contractant(s) suivant(s): DE, ES, FR, GB, IT)

1. Composé de formule: 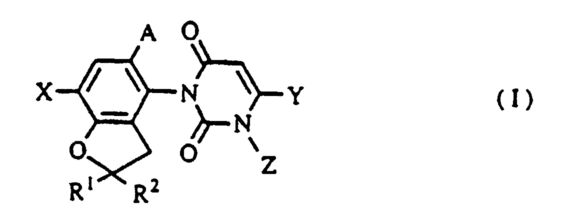 dans laquelle A est l'hydrogène, le fluor ou le chlore; X est l'hydrogène, le fluor, le chlore ou le brome; Y est un groupe méthyle éventuellement substitué par un ou plusieurs atomes d'halogène; Z est un groupe méthyle ou amino; R¹ est l'hydrogène ou un groupe alkyle en C₁-C₆, et R² est un groupe alkyle en C₁-C₆, haloalkyle en C₁-C₆, hydroxyalkyle en C₁-C₆, alcoxy(en C₁-C₆)alkyle(en C₁-C₆), alcoxy(en C₁-C₆)alcoxy (en C₁-C₆)alkyle (en C₁-C₆), acyloxy(en C₁-C₇)alkyle(en C₁-C₆), carboxyle, alcoxycarbonyle en C₁-C₆, haloalcoxycarbonyle en C₁-C₆, cycloalcoxycarbonyle en C₃-C₆, alcynyloxycarbonyle en C₂-C₆, aminocarbonyle, alkylaminocarbonyle en C₁-C₆ ou phénylamino-carbonyle ayant un groupe phényle éventuellement substitué par un groupe alkyle en C₁-C₆, alcoxy en C₁-C₆, haloalkyle en C₁-C₆, haloalcoxy en C₁-C₆, halogéno, nitro, cyano ou alkylthio C₁-C₆, à la condition que si R¹ est l'hydrogène et A est le fluor ou le chlore, X doit être différent du chlore et du brome.

2. Composé selon la revendication 1, caractérisé en ce que R¹ est un groupe alkyle en C₁-C₃.

3. Composé selon la revendication 1, caractérisé en ce que R¹ est un groupe méthyle.

4. Composé selon la revendication 1, 2 ou 3, caractérisé en ce que A est l'hydrogène.

5. Composition herbicide comprenant, comme composé actif, un composé selon l'une des revendications 1 à 4, et un support ou un diluant inerte.

6. Procédé pour exterminer des mauvaises herbes indésirables, qui comprend l'application d'un composé selon l'une des revendications 1 à 4 sur une surface où les mauvaises herbes indésirables poussent ou pousseront.

7. Utilisation d'un composé selon la revendication 1 comme herbicide.

8. Composé de formule: 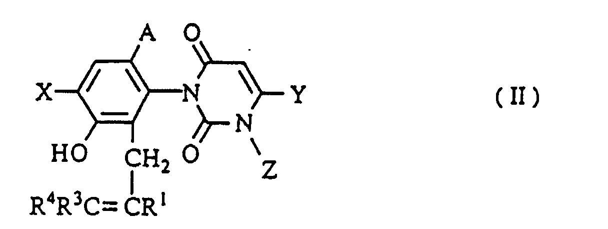 dans laquelle A est l'hydrogène, le fluor ou le chlore; X est l'hydrogène, le fluor, le chlore ou le brome; Y est un groupe méthyle éventuellement substitué par un ou plusieurs atomes d'halogène; Z est un groupe méthyle ou amino; R¹ est l'hydrogène ou un groupe alkyle en C₁-C₆; et R³ et R⁴ sont identiques ou différents et sont l'hydrogène ou un groupe alkyle en C₁-C₆ avec la condition que le nombre total d'atomes de carbone dans R³ et R⁴ ne soit pas supérieur à 6.

9. Composé de formule: 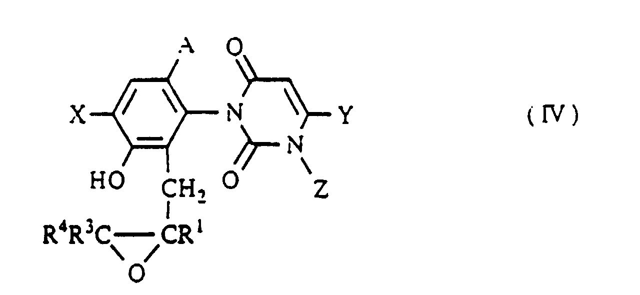 dans laquelle A est l'hydrogène, le fluor ou le chlore; X est l'hydrogène, le fluor, le chlore ou le brome; Y est un groupe méthyle éventuellement substitué par un ou plusieurs atomes d'halogène; Z est un groupe méthyle ou amino; R¹ est l'hydrogène ou un groupe alkyle en C₁-C₆; et R³ et R⁴ sont identiques ou différents et sont l'hydrogène ou un groupe alkyle en C₁-C₆ avec la condition que le nombre total d'atomes de carbone dans R³ et R⁴ ne soit pas supérieur à 6.

## Revendications (Revendications pour l'(les) Etat(s) contractant(s) suivant(s): AT, CH, LI, GR)

1. Composé de formule: 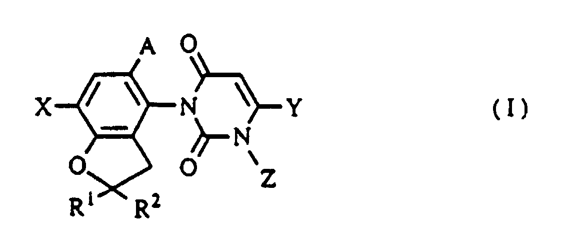 dans laquelle A est l'hydrogène, le fluor ou le chlore; X est l'hydrogène, le fluor, le chlore ou le brome; Y est un groupe méthyle éventuellement substitué par un ou plusieurs atomes d'halogène; Z est un groupe méthyle ou amino; R¹ est l'hydrogène ou un groupe alkyle en C₁-C₆; et R² est un groupe alkyle en C₁-C₆, haloalkyle en C₁-C₆, hydroxyalkyle en C₁-C₆, alcoxy(en C₁-C₆)alkyle(en C₁-C₆), alcoxy(en C₁-C₆)alcoxy(en C₁-C₆) alkyle(en C₁-C₆), acyloxy(en C₁-C₇)alkyle(en C₁-C₆), carboxyle, alcoxycarbonyle en C₁-C₆, haloalcoxycarbonyle en C₁-C₆, cycloalcoxycarbonyle en C₃-C₆, alcynyloxycarbonyle en C₂-C₆, aminocarbonyle, alkylaminocarbonyle en C₁-C₆ ou phénylaminocarbonyle ayant un groupe phényle éventuellement substitué par un groupe alkyle en C₁-C₆, alcoxy en C₁-C₆, haloalkyle en C₁-C₆, haloalcoxy en C₁-C₆, halogéno, nitro, cyano ou alkylthio C₁-C₆.

2. Composé selon la revendication 1, caractérisé en ce que R¹ est un groupe alkyle en C₁-C₃.

3. Composé selon la revendication 1, caractérisé en ce que R¹ est un groupe méthyle.

4. Composé selon la revendication 1, 2 ou 3, caractérisé en ce que A est l'hydrogène.

5. Composition herbicide comprenant, comme composé actif, un composé selon l'une des revendications 1 à 4, et un support ou un diluant inerte.

6. Procédé pour exterminer des mauvaises herbes indésirables, qui comprend l'application d'un composé selon l'une des revendications 1 à 4 sur une surface où les mauvaises herbes indésirables poussent ou pousseront.

7. Utilisation d'un composé selon la revendication 1 comme herbicide.

8. Composé de formule: 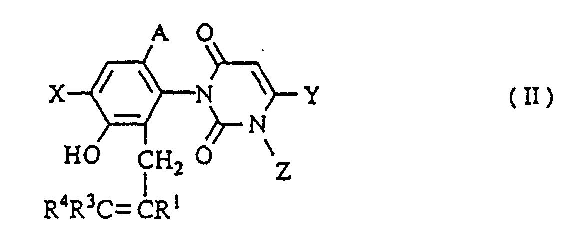 dans laquelle A est l'hydrogène, le fluor ou le chlore; X est l'hydrogène, le fluor, le chlore ou le brome; Y est un groupe méthyle éventuellement substitué par un ou plusieurs atomes d'halogène; Z est un groupe méthyle ou amino; R¹ est l'hydrogène ou un groupe alkyle en C₁-C₆; et R³ et R⁴ sont identiques ou différents et sont l'hydrogène ou un groupe alkyle en C₁-C₆ avec la condition que le nombre total d'atomes de carbone dans R³ et R⁴ ne soit pas supérieur à 6.

9. Composé de formule: 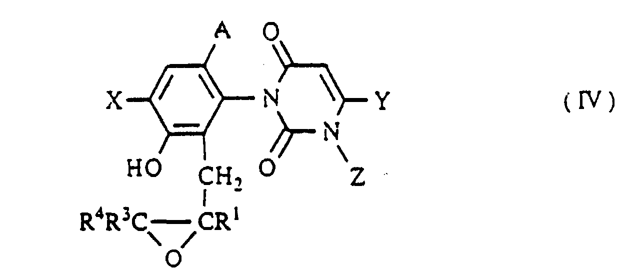 dans laquelle A est l'hydrogène, le fluor ou le chlore; X est l'hydrogène, le fluor, le chlore ou le brome; Y est un groupe méthyle éventuellement substitué par un ou plusieurs atomes d'halogène; Z est un groupe méthyle ou amino; R¹ est l'hydrogène ou un groupe alkyle en C₁-C₆; et R³ et R⁴ sont identiques ou différents et sont l'hydrogène ou un groupe alkyle en C₁-C₆ avec la condition que le nombre total d'atomes de carbone dans R³ et R⁴ ne soit pas supérieur à 6.
